# EUROPEAN PATENT APPLICATION

(11) **EP 4 714 497 A2**
(43) Date of publication of application: **25.03.2026**
(21) Application number: 25220465.6
(22) Date of filing: 10.01.2023
(51) Int. Cl.: A61P 25/28

(54) **TREATMENT OF GM2 GANGLIOSIDOSIS**

(30) Priority: 10.01.2022 EP 22150783; 31.08.2022 EP 22193136
(62) Divisional of application: 23700690.3
(71) Applicant: Azafaros B.V., 2333 CH Leiden (NL)
(72) Inventor: ROBIN, Jennifer, 2333 CH Leiden (NL); BLATTER, Fritz, 2333 CH Leiden (NL); HETT, Robert, 2333 CH Leiden (NL); LANDSKRONER, Kyle, 4052 Basel (CH)
(74) Representative: Boult Wade Tennant LLP

(57) **Abstract**

The invention relates to a compound of formula (I), 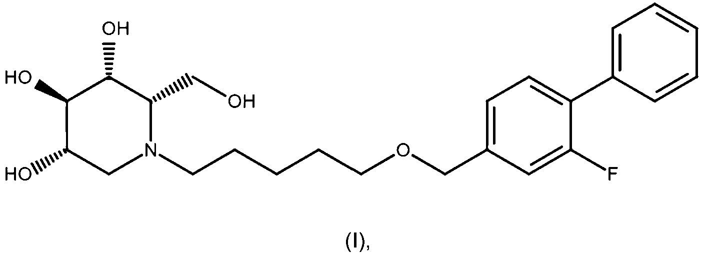 for use in treating GM2 gangliosidosis, specifically Sandhoff disease or Tay-Sachs disease. Specifically, the invention relates to a compound of formula (I) for use in increasing survival of a subject suffering from GM2 gangliosidosis, specifically Sandhoff disease or Tay-Sachs disease. The compound of formula (I) may also be used as an anti-inflammatory agent.

## Description

### FIELD OF THE INVENTION

The invention relates to a compound of formula (I), 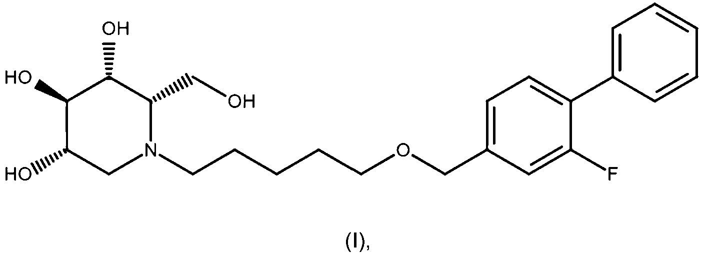 for use in treating GM2 gangliosidosis, specifically Sandhoff disease or Tay-Sachs disease. Specifically, the invention relates to a compound of formula (I) for use in increasing survival of a subject suffering from GM2 gangliosidosis, specifically Sandhoff disease or Tay-Sachs disease.

### BACKGROUND OF THE INVENTION

GM2 gangliosidoses are a group of degenerative/inflammatory diseases affecting the brain caused by a deficiency of different proteins needed to break down lipids. Examples of a GM2 gangliosidosis include Sandhoff disease and Tay-Sachs disease. GM2 gangliosidoses are characterized by rapid neurological deterioration and typically death before 4 years of age since treatment success has been limited to date. They are a result of the inheritance of defects in genes responsible for encoding the enzymes required to breakdown GM2 ganglioside (a lipid). This lipid then accumulates in the body, reaching its highest levels in the brain where it is most abundant. In their severe infantile form, these diseases become evident at about 6 months of age. Juvenile- and adult-onset forms of the diseases are associated with a wide range of neurological symptoms, many of which are debilitating and ultimately lethal.

GM2 ganglioside is normally degraded in a cell's lysosomes through the concerted action of the products of three genes, HEXA, HEXB, and GM2A. A defect in any one of these genes can result in a deficiency of HEXA activity towards GM2 ganglioside, which then cannot be broken down. The most common form of GM2 gangliosidosis is Tay-Sachs disease, which results from mutations in HEXA. Mutations in HEXB, encoding the beta subunit of β-hexosaminidase, result in Sandhoff disease. Approximately 1 in 25 individuals of Ashkenazi Jewish descent is a carrier for Tay-Sachs disease, but carrier screening has successfully reduced the incidence in that population. The prevalence of Sandhoff disease is approximately 1 in 384,000 live births, but is increased in certain populations.

Mouse models of Tay-Sachs and Sandhoff diseases have been created by targeted disruptions of the mouse Hexa and Hexb genes. Mutations targeting Hexb gene result in a severe neurological phenotype characterized by extensive GM2 ganglioside accumulation in the brain and spinal cord, leading to spasticity, muscle weakness, rigidity, and ultimately death by 16-17 weeks of age (Gulinello et al., (2008), Behavioural Brain Research: 193, 315-319). These mice are used extensively as a model for studying potential therapies for the GM2 gangliosidoses.

In addition to the genetic cause of GM2 gangliosidoses, an inflammatory response (microglial activation, macrophage infiltration, oxidative damage) has been associated with these lysosomal storage diseases. Specifically, inflammation is known to result from excess GM2 storage in the brain and conversely, chronic inflammation of the brain has been implicated in the direct as well as indirect disease pathogenesis and progression in GM2 gangliosidoses (Jeyakumar et al., (2003), Brain: 126, 974-987). It was also demonstrated that production of the inflammatory cytokines TNFα, IL1β and TGFβ1 was elevated in brains of Sandhoff mice, but not in control mice. Later studies confirmed these findings in humans affected with GM2 gangliosidoses. Finally, macrophage activation and rise in the level of an inflammatory cytokine, Macrophage inflammatory protein-1α (MIP-1α), has been implicated in pathogenesis of Sandhoff disease.

Further evidence for a role of inflammation in GM2 has been demonstrated by the use of anti-inflammatory drug, Aspirin, which has been shown to significantly slow down the progression of disease in Sandhoff mice (Jeyakumar et al., (2004), Ann. Neurol.: 56, 642-649). When Aspirin was combined with substrate reduction therapy (treatment with compounds which inhibit the synthesis of the lipids involved in GM2 gangliosidoses) synergy was found to result (11%, improvement in survival) with a maximum improvement of 73% survival.

Another therapy for GM2 gangliosidosis that has recently shown promise is the drug pyrimethamine. Treatment of GM2 gangliosidosis cell lines with pyrimethamine demonstrated improved enzyme activity and has led to some success in initial human trials (Ashe et al., (2011), PLOS One: 6, e21758). Pyrimethamine is an approved drug for treating malaria; however, pyrimethamine has also been shown to enhance production of hexosaminidase (Maegawa et al., (2007), J. Biol. Chem.: 282, 9150-9161).

Substrate reduction therapy (SRT), in which the primary pharmacological action of the therapeutic agents used to treat GM2 gangliosidoses is to inhibit the formation of glucosylceramide (GlcCer) by inhibiting the enzyme glucosylceramide synthase (GCS), and subsequently decreasing biosynthesis of more complex glycosphingolipids (GSL) is an additional therapeutic principle. SRT has been approved for Gaucher type 1 and Niemann Pick type C diseases.

Miglustat (N-butyldeoxynojirimycin), which is an iminosugar (a synthetic analogue of D-glucose) that was developed as an inhibitor of glucosylceramide synthase (also called ceramide glucosyltransferase, EC 2.4.1.80, UniProt code: Q16739) exhibits a large volume of distribution and has the capacity to access deep organs such as the brain, bone and lung. In addition, miglustat has been shown to have anti-inflammatory properties. However, clinically, miglustat has not shown to be therapeutically effective for juvenile GM2 gangliosidosis (Maegawa et al. (2009), Molecular Genetics and Metabolism: 98, 215-224).

WO2015/147639 A1 describes a wide range of derivatives of deoxynojirimycin, including a compound of a formula (referred to as compound of formula (I) herein):

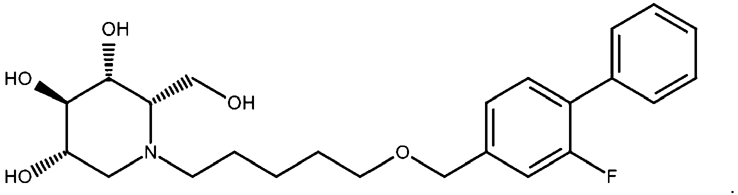

Compound of formula (I) is a potent dual inhibitor of glucosylceramide synthase and non-lysosomal glucosylceramidase (GBA2, UniProt code: Q9HCG7).

In view of a very low survival rate and short life expectancy of patients with GM2 gangliosidoses such as Sandhoff disease or Tay-Sachs disease, a need exists for a new therapy which would increase survival of patients suffering from such diseases.

### SUMMARY OF THE INVENTION

The inventors have tested the compound of formula (I) in a mouse model of GM2 gangliosidosis (specifically Sandhoff disease) and found that this compound is very effective in extending survival of diseased mice compared to vehicle (placebo) treated mice. Moreover, treatment using the compound was shown to significantly improve motor function in treated mice. Accordingly, in a first aspect, the invention provides a compound of formula (I), 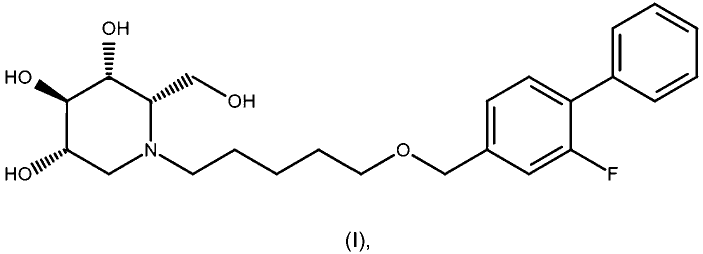 for use in improving motor function of a subject, in particular a subject suffering from GM2 gangliosidosis, specifically Sandhoff disease or Tay-Sachs disease. Preferably, the subject suffers from Sandhoff disease. The improved motor function improves the quality of life of the subject.

In a further aspect, the invention provides a compound of formula (I), 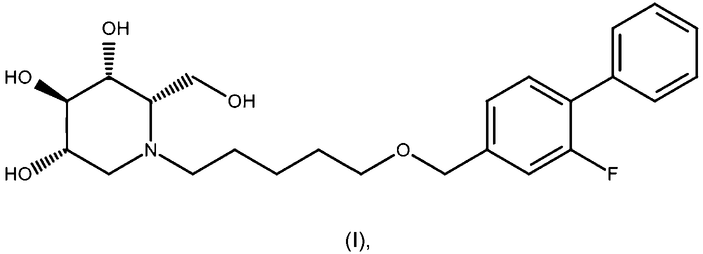 for use in treating a GM2 gangliosidosis.

In a further aspect, the invention provides a compound of formula (I), 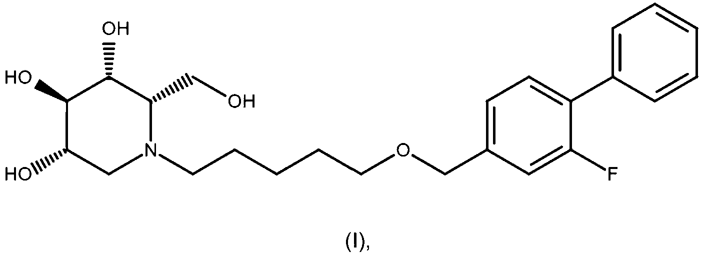 for use in treating Sandhoff disease.

In a further aspect, the invention provides a compound of formula (I), 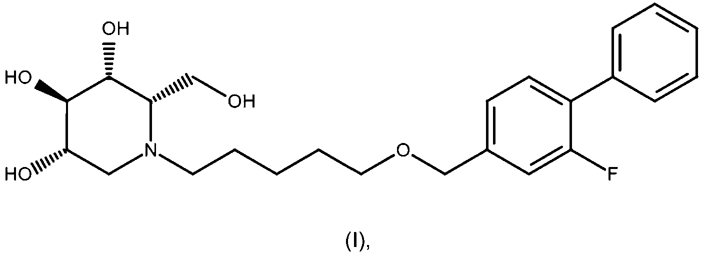 for use in treating Tay-Sachs disease.

In a further aspect, the invention provides a compound of formula (I), 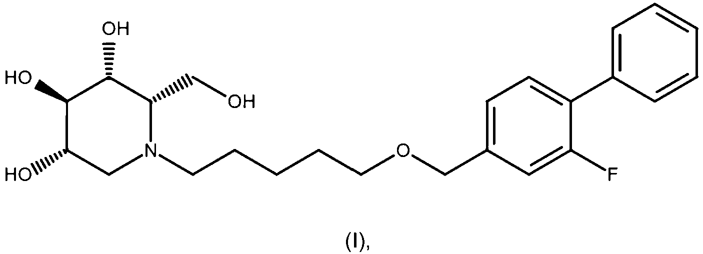 for use in eliminating, alleviating or ameliorating symptoms of GM2 gangliosidosis, in particular Sandhoff disease or Tay-Sachs disease. Preferably, the compound of formula (I) eliminates, alleviates or ameliorates symptoms of Sandhoff disease.

In a further aspect, the invention provides a compound of formula (I), 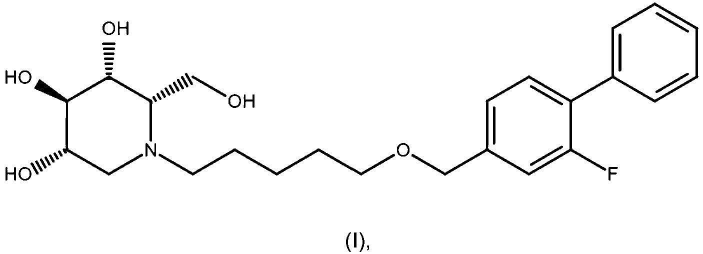 for use in extending life or life expectancy of a subject suffering from GM2 gangliosidosis, in particular Sandhoff disease or Tay-Sachs disease, preferably Sandhoff disease.

In a further aspect, the invention provides a compound of formula (I), 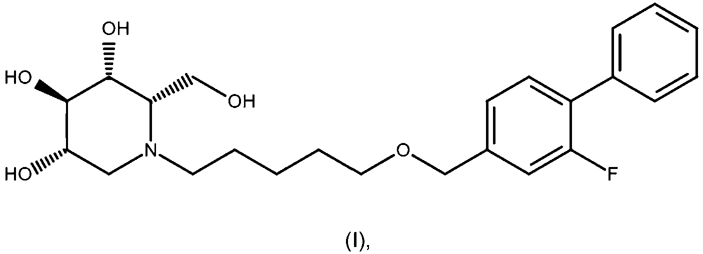 for use in increasing survival of a subject suffering from a GM2 gangliosidosis.

In a further aspect, the invention provides a compound of formula (I), 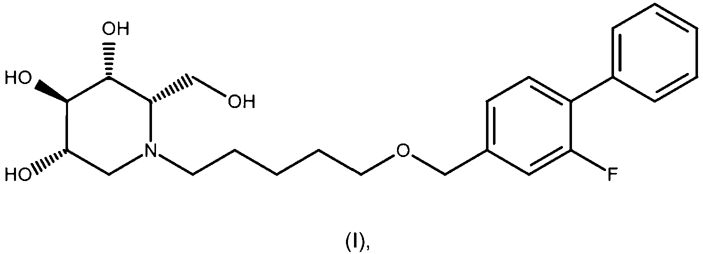 for use in increasing survival of a subject suffering from Sandhoff disease.

In a further aspect, the invention provides a compound of formula (I), 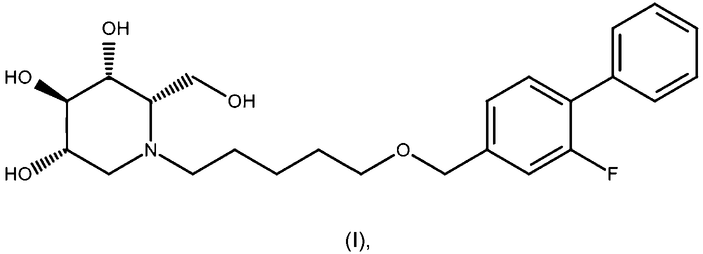 for use in increasing survival of a subject suffering from Tay-Sachs disease.

The medical uses of the invention may be expressed in any suitable form. Thus, in a further aspect, the invention provides a method of treating GM2 gangliosidosis, in particular Sandhoff disease or Tay-Sachs disease, the method comprising administering to the subject suffering from GM2 gangliosidosis, in particular Sandhoff disease or Tay-Sachs disease a compound of formula (I): 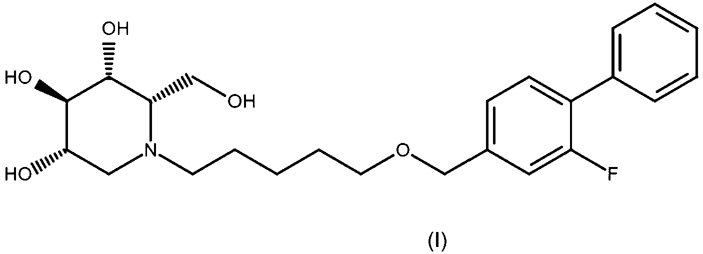 in an amount effective to increase survival of the subject.

Preferably, the method treats Sandhoff disease. Thus, the invention provides a method of treating Sandhoff disease, the method comprising administering to the subject suffering from Sandhoff disease a compound of formula (I) in an amount effective to increase survival of the subject.

In a further aspect, the invention provides a method of treating Tay-Sachs disease, the method comprising administering to the subject suffering from Tay-Sachs disease a compound of formula (I): 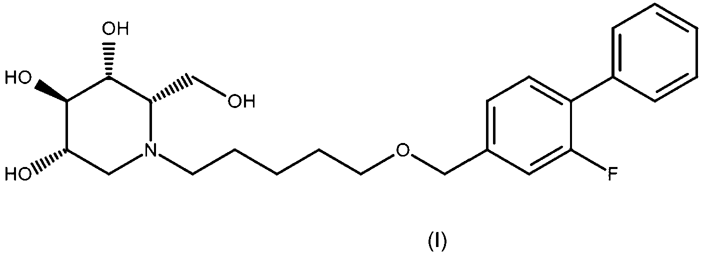 in an amount effective to increase survival of the subject.

In a further aspect, the invention provides a method of increasing survival of a subject suffering from GM2 gangliosidosis, in particular Sandhoff disease or Tay-Sachs disease, the method comprising administering to the subject suffering from GM2 gangliosidosis, in particular Sandhoff disease or Tay-Sachs disease a compound of formula (I): 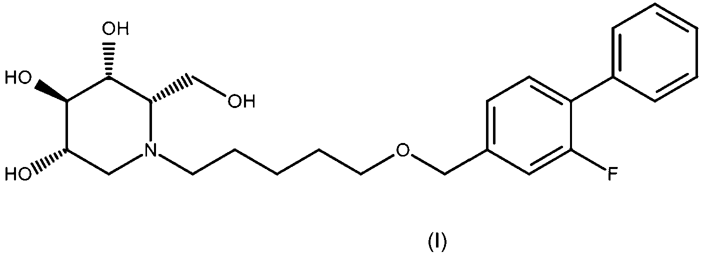 in an amount effective to increase survival of the subject.

Preferably, the subject is suffering from Sandhoff disease. Thus, the invention provides a method of increasing survival of a subject suffering from Sandhoff disease, the method comprising administering to the subject suffering from Sandhoff disease a compound of formula (I): 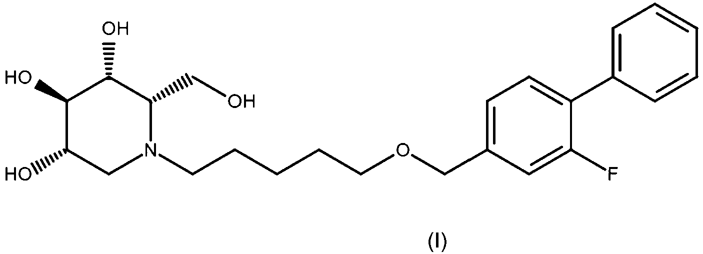 in an amount effective to increase survival of the subject.

In a further aspect, the invention provides a method of increasing survival of a subject suffering from Tay-Sachs disease, the method comprising administering to the subject suffering from Tay-Sachs disease a compound of formula (I): 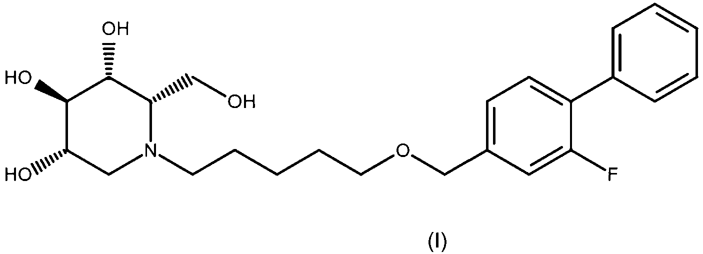 in an amount effective to increase survival of the subject.

In a further aspect, the invention provides a method of treating a subject suffering from Sandhoff disease or Tay-Sachs disease, wherein the method increases survival of the subject, the method comprising administering to the subject suffering from Sandhoff disease or Tay-Sachs disease a compound of formula (I) 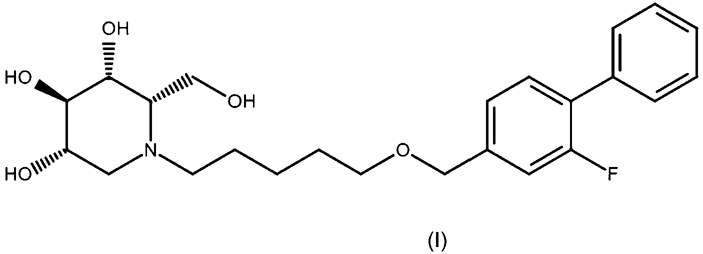 in an amount effective to increase survival of the subject.

In a further aspect, the invention provides a use of a compound of formula (I) 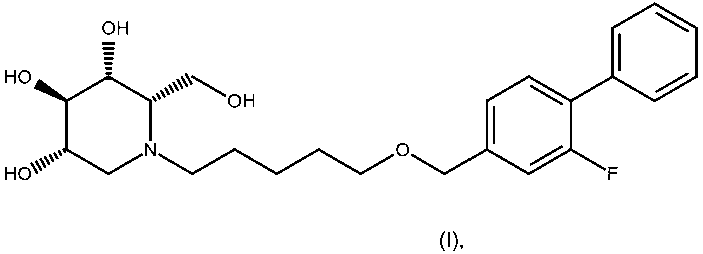 for treating a subject suffering from GM2 gangliosidosis, in particular Sandhoff disease or Tay-Sachs disease. Preferably, the subject is suffering from Sandhoff disease.

In a further aspect, the invention provides a use of a compound of formula (I) 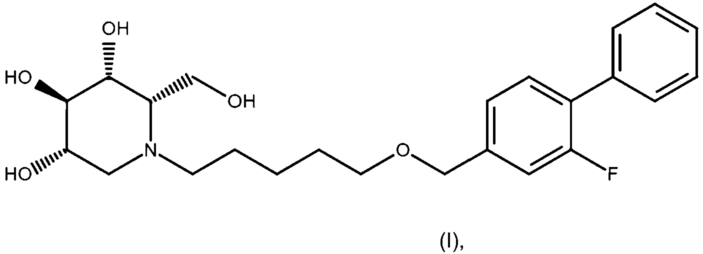 for increasing survival of a subject suffering from GM2 gangliosidosis, in particular Sandhoff disease or Tay-Sachs disease. Preferably, the subject is suffering from Sandhoff disease.

In a further aspect, the invention provides a use of a compound of formula (I) 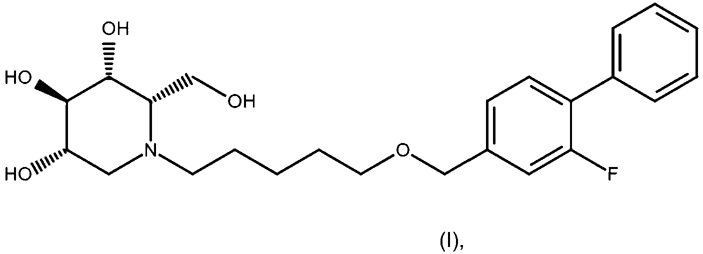 for producing a medicament for increasing survival of a subject suffering from GM2 gangliosidosis, in particular Sandhoff disease or Tay-Sachs disease. Preferably, the subject is suffering from Sandhoff disease.

In a further aspect, the invention provides a compound of formula (I), 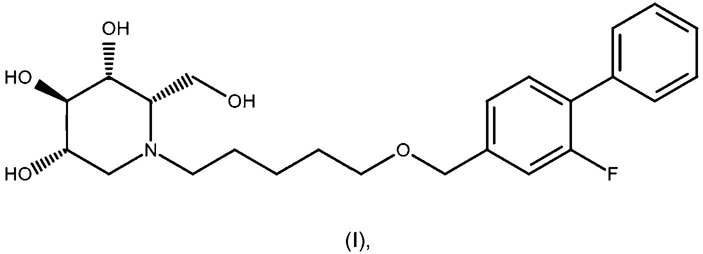 for use as an anti-inflammatory agent, preferably in a subject suffering from a GM2 gangliosidosis. The compound may exert its anti-inflammatory effect by downregulation pro-inflammatory genes, such as ITGAX, TREM2, and CXCL10.

The invention also provides a use of a compound of formula (I), 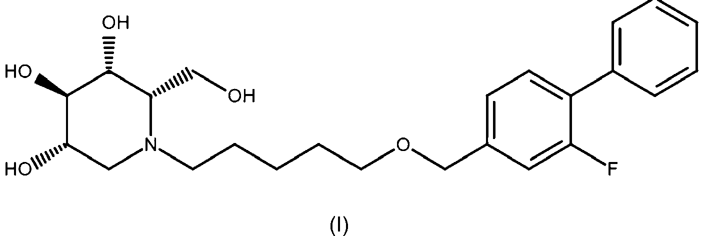 in the manufacture of a medicament for increasing survival of a subject suffering from GM2 gangliosidosis, in particular Sandhoff disease or Tay-Sachs disease, preferably Sandhoff disease.

The invention also provides a use of a compound of formula (I), 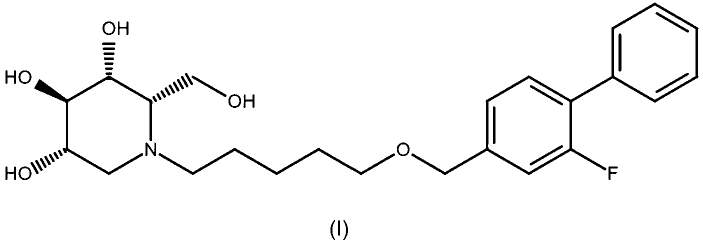 in the manufacture of an anti-inflammatory medicament.

For any of the aspects described herein, whilst the disease is generally a GM2 gangliosidosis the disease is typically Sandhoff disease or Tay Sachs. The subject is preferably suffering from Sandhoff disease according to all aspects.

For any of the aspects described above, the compound of formula (I) is preferably in the crystalline form. Preferably, the crystalline form is 'Form 3' crystalline form, which displays a reflection, stated as a 2Θ value, at 17.8 ± 0.2°, in an X-ray powder diffraction pattern, wherein the reflection at 17.8 ± 0.2° is one of the four strongest reflections in the X-ray powder diffraction pattern.

Preferably, for any of the aspects described above, the compound is administered to the subject as a pharmaceutical composition. The pharmaceutical composition may comprise the compound together with at least one pharmaceutically acceptable carrier, diluent or excipient.

Other preferred embodiments of the compound for use according to the invention appear throughout the specification and in particular in the examples.

As discussed above, the present inventors have surprisingly discovered that compound of formula (I) increases survival (i.e. life expectancy) of a subject suffering from GM2 gangliosidosis (based on a Sandhoff disease mice model). In addition, the inventors of the present application have discovered that compound of formula (I) significantly improves motor skills and/or positively impacts the behaviour assessment of a subject, in particular a subject suffering from GM2 gangliosidosis (e.g. Sandhoff disease).

Each aspect or embodiment as defined herein may be combined with any other aspect(s) or embodiment(s) unless clearly indicated to the contrary. In particular any feature indicated as being preferred or advantageous may be combined with any other feature or features indicated as being preferred or advantageous.

### BRIEF DESCRIPTION OF THE DRAWINGS

These and other aspects of the invention will now be described with reference to the accompanying Figures, in which:

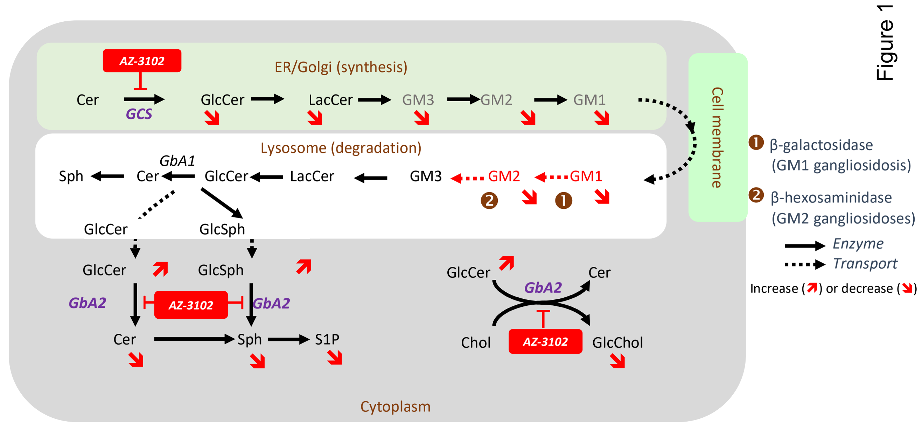

Figure 1 summarizes the mode of action of the crystalline form of compound of formula (I) (AZ-3102).
Figure 2A shows the X-ray powder diffraction pattern of 'Form 3' crystalline form of the compound of formula (I), obtained by equilibration with acetonitrile.
Figure 2B shows an overlay of the X-ray powder diffraction patterns of 'Form 3' crystalline form of the compound of formula (I), obtained by equilibration with TBME, water, isopropanol, ethyl acetate, acetonitrile, or anisole, respectively.
Figure 3 shows an overlay of the X-ray powder diffraction patterns of two crystalline forms of compound of formula (I) obtained by equilibration with acetonitrile:
   1) 'Form 3', and
   2) 'Form 2'.
Figure 4 shows a schematic description of study protocols in Sandhoff mice models.
Figure 5 shows results of the survival study in Sandhoff mice models treated with AZ-3102 (compound (I)) and placebo (vehicle).
Figure 6 illustrates results for the Open Field Test (OFT) assessment in Sandhoff mice models treated with AZ-3102 (compound (I)) and placebo (vehicle).
Figure 7 shows results for the rotarod test in Sandhoff mice models treated with AZ-3102 (compound (I)) and placebo (vehicle).
Figure 8 illustrates plasma and brain concentration versus time profiles for AZ-3102 following 16-weeks of once daily administration.
Figure 9 illustrates average brain to plasma ratios in the cerebellum at 2, 4, and 24h post steady-state dosing.
Figure 10 illustrates GlcCer C16:0 and C18:0 concentration versus time profiles in the cerebellum.
Figure 11 illustrates survival of Hexb (-/-) mice at lower doses of AZ-3102.
Figure 12 illustrates gene expression from RNA-sequence data for midbrain section. The data is presented as fold expression versus vehicle treated Het mice.
Figure 13 illustrates the effect of AZ-3102 on production of Glial fibrillary acidic protein (GFAP).

### DETAILED DESCRIPTION OF THE INVENTION

Unless otherwise defined herein, scientific, and technical terms used in connection with the present invention shall have the meanings that are commonly understood by those of ordinary skill in the art. The meaning and scope of the terms should be clear, however, in the event of any latent ambiguity, definitions provided herein take precedent over any dictionary or extrinsic definition.

It should be understood that singular prepositions such as "a," "an," and "the," are often used for convenience, however, all instances of the singular are intended to encompass the plural unless otherwise indicated either explicitly or from context. The terms "comprising", "including" and "having" are intended to be inclusive and mean that there may be additional elements other than the listed elements. Further, it should be understood that all references, including journal articles, books, patents, technical documents, and the like, mentioned in this disclosure are hereby incorporated by reference in their entirety and for all purposes.

The term "about" as used herein for numerical data refers to a value within 10% of the underlying parameter (i.e., plus or minus 10%), and use of the term "about" at the beginning of a string of values modifies each of the values (i.e., "about 1, 2 and 3" refers to about 1, about 2 and about 3). For example, a temperature of "about 85 °C" can include temperatures between 75 °C and 95 °C.

The term "melting point" is well known in the art. The term "relatively high melting point" as used herein is intended to encompass crystalline forms that are stable enough to be formulated as pharmaceutical compositions.

The term "composition" as used herein is intended to encompass a product comprising the specified ingredients in the specified amounts, as well as any product which results, directly or indirectly, from combination of the specified ingredients in the specified amounts. Such term in relation to a pharmaceutical composition is intended to encompass a product comprising the compound of formula (I) and/or the crystalline form of compound of formula (I), and optionally additional ingredients that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition comprising a compound and/or a crystalline form of the present invention, and optionally a pharmaceutically acceptable carrier, diluent or excipient. By "pharmaceutically acceptable" it is meant the carrier, diluent or excipient (if present) must be compatible with the other ingredients of the formulation and not deleterious to the recipient thereof.

The terms "therapeutically effective amount" and "amount effective to" deliver the effect intend to encompass the amount of a compound, a crystalline form or a pharmaceutical composition that, when administered to a patient for treating a disease, is sufficient to effect such treatment for the disease. The terms "therapeutically effective amount" and "amount effective to" deliver the effect intend to encompass the amount of a compound, a crystalline form or a pharmaceutical composition that, when administered to a patient for delivering a therapeutic effect (e.g. increasing survival), is sufficient to achieve such therapeutic effect. The therapeutically effective amount will vary depending on the disease and its severity, and/or the age and weight of the patient. The term "subject" (or "patient") includes, but is not limited to, animals such as, for example, mammals. Preferably, the subject is a human.

The present invention provides a compound of formula (I), 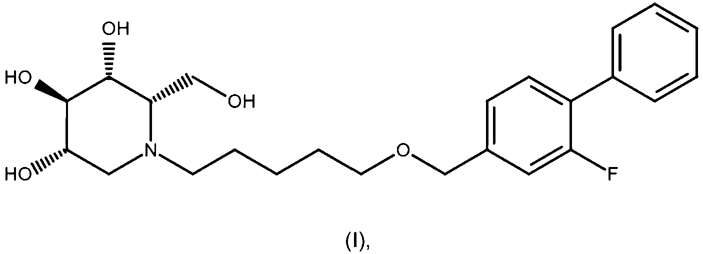 for use in improving motor function of a subject.

The subject may suffer from a disease involving abnormal levels of glucosylceramide and/or higher levels of glycosphingolipids. Preferably, the subject suffers from GM2 gangliosidosis, in particular Sandhoff disease or Tay-Sachs disease. More preferably still, the subject suffers from Sandhoff disease.

A method of improving motor function of a subject is also provided, the method comprising administering to the subject a compound of formula (I) in an amount effective to improve motor function of the subject. Preferably, the subject suffers from GM2 gangliosidosis, in particular Sandhoff disease or Tay-Sachs disease. More preferably still, the subject suffers from Sandhoff disease.

The invention also provides a compound of formula (I), 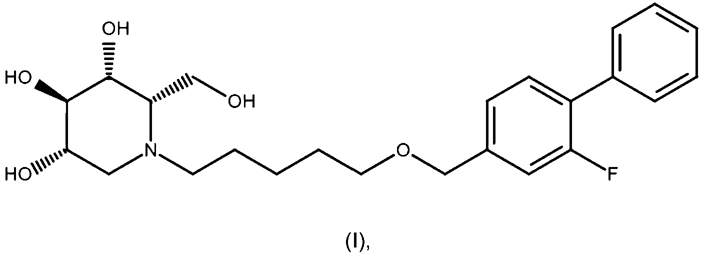 for use in treating GM2 gangliosidosis, in particular Sandhoff disease or Tay-Sachs disease.

Preferably, the compound of formula (I) is used to treat Sandhoff disease.

The invention provides a compound of formula (I), 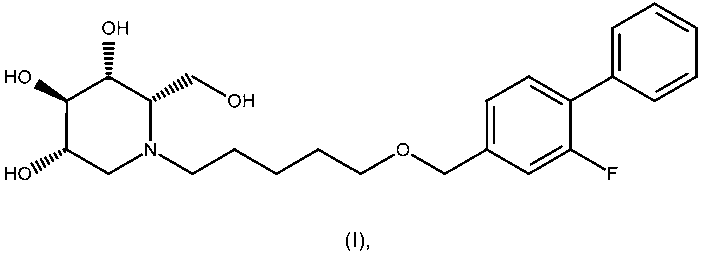 for use in eliminating, alleviating or ameliorating symptoms of GM2 gangliosidosis, in particular Sandhoff disease or Tay-Sachs disease. Preferably, the compound of formula (I) is used to eliminate, alleviate or ameliorate symptoms of Sandhoff disease.

As shown in the Examples, treatment of subjects suffering from Sandhoff disease with the compound of formula (I) eliminated, alleviated or ameliorated certain symptoms of Sandhoff disease. For example, motor function of the subject has been improved.

It will be appreciated that the treatment of Sandhoff disease may result in the elimination, alleviation or amelioration of one or more symptoms of Sandhoff disease and need not necessarily eliminate, alleviate or ameliorate every symptom of Sandhoff disease that the patient experienced prior to treatment.

Since clinical presentation of Sandhoff disease and Tay-Sachs disease is relatively indistinguishable and the biochemical cause of both diseases is similar (Tay-Sachs disease results from beta-hexosaminidase A deficiency while Sandhoff disease is associated with beta-hexosaminidase A and/or beta-hexosaminidase B deficiency), the subject suffering from Tay-Sachs disease is also expected to experience elimination, alleviation or amelioration of certain symptoms associated with Tay-Sachs disease upon administration of the compound of formula (I) (or crystalline form thereof).

By 'elimination' of a symptom is meant that the subject essentially no longer experiences that symptom. By 'alleviation' of a symptom is meant that the subject experiences a significant improvement in the symptom. The alleviation is preferably from severe to mild. By 'amelioration' of a symptom is meant that the subject experiences a modest improvement in the symptom.

Thus, in particular embodiments, the treatment, use and the like provided herein may result in the elimination, alleviation or amelioration of one or more symptoms of Sandhoff disease or Tay-Sachs disease. For example, one or more symptoms may be alleviated and one or more different symptoms may be ameliorated.

The symptoms of Sandhoff disease include at least one of muscle weakness, decreased movement, loss of motor skills, increased reaction to loud noises, seizures, vision loss, hearing loss, intellectual disability, an eye abnormality, enlarge organs, bone abnormalities, speech difficulties, loss of cognitive function, loss of muscle coordination, and/or psychiatric problems. The compound of formula (I) may be used to alleviate, eliminate and/or ameliorate at least one symptom of Sandhoff disease in a subject suffering from Sandhoff disease. Preferably, the compound of formula (I) alleviates, eliminates and/or ameliorates at least 2, at least 3, at least 4, or at least 5 symptoms of Sandhoff disease. For example, the compound of formula (I) may improve motor skills and muscle coordination in a subject.

The above symptoms are also presented by subjects suffering from Tay-Sachs disease. Thus, any embodiment described in the context of the Sandhoff disease may also be applied to Tay-Sachs disease, or indeed to GM2 gangliosidoses more generally.

Thus, alternatively viewed, provided is a method of eliminating, alleviating and/or ameliorating one or more of the above symptoms by administering a therapeutically effective amount of the compound of formula (I) to a subject in need thereof. The subject may suffer from GM2 gangliosidosis, in particular Sandhoff disease or Tay-Sachs disease.

In addition, the invention provides a compound of formula (I), 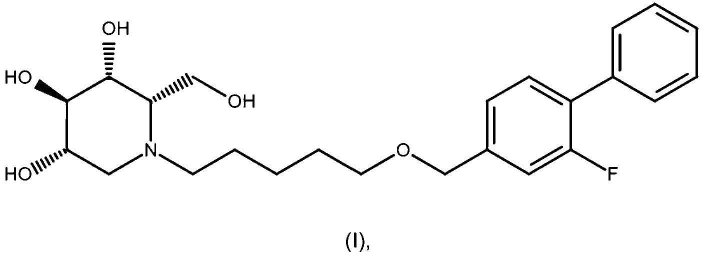 for use in extending life of a subject suffering from GM2 gangliosidosis, in particular Sandhoff disease or Tay-Sachs disease. Preferably, the compound of formula (I) (or the crystalline form thereof) is used to extend life of a subject suffering from Sandhoff disease.

A method of extending life of a subject suffering from GM2 gangliosidosis, in particular Sandhoff disease or Tay-Sachs disease is also provided, the method comprising administering to the subject a compound of formula (I) in an amount effective to extend the life of a subject suffering from GM2 gangliosidosis, in particular Sandhoff disease or Tay-Sachs disease.

The life expectancy of a subject suffering from GM2 gangliosidosis, in particular Sandhoff disease or Tay-Sachs disease is very short, especially in the infant-form of the disease. The inventors of the present application have surprisingly discovered that compound of formula (I) (or crystalline form thereof) increases life expectancy by at least 22% in mice model (treated vs. untreated sample).

Thus, the compound of formula (I) (or crystalline form thereof, preferably in 'Form 3') may be used to extend the life of a subject (or life expectancy of a subject) by at least 5%, at least 10%, at least 15%, at least 20%, at least 25%, at least 30%, at least 35%, at least 40%, at least 45%, at least 50%, at least 55%, or at least 60% as compared to the life (or life expectancy) the subject would have without treatment with compound of formula (I). In other words, the compound of formula (I) (or the crystalline form thereof, preferably in 'Form 3') may be used to extend the life of a subject (or life expectancy of a subject) by at least 1 month, at least 3 months, at least 6 months, at least 9 months, at least 1 year, at least 2 years, at least 3 years, at least 4 years, at least 5 years, at least 6 years, at least 7 years, at least 8 years, at least 9 years, at least 10 years, at least 11 years, at least 12 years, at least 13 years, at least 14 years, at least 15 years, at least 16 years, at least 17 years, at least 18 years, at least 19 years, at least 20 years, at least 25 years, at least 30 years, at least 35 years, at least 40 years, at least 45 years, or at least 50 years as compared to the life (or life expectancy) the subject would have without treatment with the compound of formula (I) (or the crystalline form thereof, preferably in 'Form 3').

The invention provides a compound of formula (I), 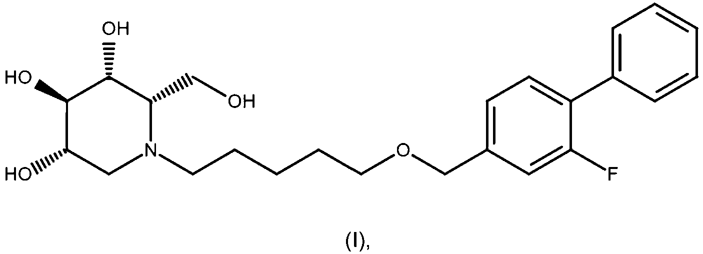 for use in increasing survival of a subject suffering from GM2 gangliosidosis, in particular Sandhoff disease or Tay-Sachs disease, preferably Sandhoff disease. Preferably, the compound of formula (I) is in a crystalline form (preferably a 'Form 3' crystalline form).

The compound of formula (I) (or the crystalline form thereof) may have a reduced clearance in the brain as compared to plasma. This makes the compound of formula (I) (or the crystalline form thereof) particularly suitable for use in treating a GM2 gangliosidosis. For example, the concentration of the compound of formula (I) (or the crystalline form thereof) in the brain may be maintained for at least 12 hours, at least 24 hours, or at least 48 hours. That is to say that the concentration of the compound of formula (I) (or the crystalline form thereof) in the brain does not (substantially) change for at least 12 hours, at least 24 hours, or at least 48 hours after the administration (once the brain concentration of the compound of formula (I) or the crystalline form thereof reaches a steady state, or a maximum value based on the dose administered).

The lethality of subjects suffering from Sandhoff disease is very high, especially in the infant-form of the disease. The present inventors have unexpectedly discovered that administration of compound of formula (I) (or crystalline form thereof, preferably in 'Form 3') to subjects suffering from Sandhoff disease (HEXB mutation) increases survival of the subjects even at a very small dose of the administered compound. By "increases survival" is meant an improvement in survival time compared to the situation in which the compound was not administered. They survive for significantly longer than if untreated. Thus, the compound of formula (I) (or th crystalline form thereof, preferably in 'Form 3') may be used to increase survival time of a subject (or life expectancy of a subject) by at least 5%, at least 10%, at least 15%, at least 20%, at least 25%, at least 30%, at least 35%, at least 40%, at least 45%, at least 50%, at least 55%, or at least 60% as compared to the survival time the subject would have without treatment with compound of formula (I). In other words, the compound of formula (I) (or the crystalline form thereof, preferably in 'Form 3') may be used to increase survival time of a subject by at least 1 month, at least 3 months, at least 6 months, at least 9 months, at least 1 year, at least 2 years, at least 3 years, at least 4 years, at least 5 years, at least 6 years, at least 7 years, at least 8 years, at least 9 years, at least 10 years, at least 11 years, at least 12 years, at least 13 years, at least 14 years, at least 15 years, at least 16 years, at least 17 years, at least 18 years, at least 19 years, at least 20 years, at least 25 years, at least 30 years, at least 35 years, at least 40 years, at least 45 years, or at least 50 years as compared to the survival time the subject would have without treatment with compound of formula (I) (or crystalline form thereof).

A method of increasing survival of a subject suffering from GM2 gangliosidosis, in particular Sandhoff disease or Tay-Sachs disease is also provided, the method comprising administering to the subject a compound of formula (I) (or crystalline form thereof, preferably in 'Form 3') in an amount effective to increase survival of a subject suffering from GM2 gangliosidosis, in particular Sandhoff disease or Tay-Sachs disease.

A method of treating a subject suffering from GM2 gangliosidosis, in particular Sandhoff disease or Tay-Sachs disease is also provided, wherein the method increases survival of the subject, the method comprising administering to the subject suffering from GM2 gangliosidosis, in particular Sandhoff disease or Tay-Sachs disease a compound of formula (I) (or a crystalline form thereof, preferably in 'Form 3') in an amount effective to increase survival of the subject.

Use of a compound of formula (I) (or a crystalline form thereof, preferably in 'Form 3') for increasing survival of a subject suffering from GM2 gangliosidosis, in particular Sandhoff disease or Tay-Sachs disease is also provided.

The invention provides a compound of formula (I), 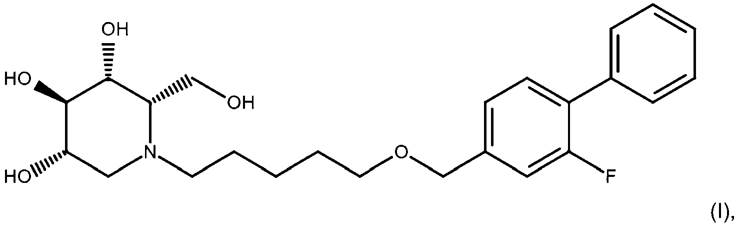 for use as an anti-inflammatory agent, preferably in a subject suffering from a GM2 gangliosidosis.

For the avoidance of doubt, all embodiments specified herein are not limited to one particular wording to define the treatment and apply *mutatis mutandis* to the medical uses or methods of treatment aspects of the invention.

The methods and uses described herein in the context of Sandhoff disease can also be applied to GM2 gangliosidoses generally, in particular to Tay-Sachs disease.

According to all aspects of the invention described herein, preferably, compound of formula (I) is in a crystalline form. The crystalline form of compound of formula (I) may be in any crystal state. The crystalline form of compound of formula (I) may be a crystalline salt or a crystalline free base (nonionized form). Preferably the crystalline form of compound of formula (I) is a crystalline free base. In addition, compound of formula (I) may form a co-crystal.

If the crystalline form of compound of formula (I) is a crystalline salt, the molecular structure of compound of formula (I) herein comprises a protonated nitrogen atom.

All aspects of the invention described herein are not limited to a single crystalline form of compound of formula (I). Solid materials may exist in more than one crystalline form. These alternative crystalline forms are termed polymorphs. Each polymorph has different orientations and/or conformations of molecules in the crystal lattice. Each crystal state, or "polymorph", exhibits a unique set of physicochemical properties due to differences in crystal structure.

Polymorphic forms may have different mechanical properties, such as fluidity and compressibility, which affect the technological properties of the compound. Stability and duration of storage of the compound may also depend on the polymorph.

Polymorphs can be distinguished from each other in different ways. Polymorphs clearly exhibit spectroscopic properties, and they can be determined using, for example, infrared spectroscopy, Raman spectroscopy, and ¹³C-NMR spectroscopy. In view of the fact that each crystalline form refracts X-rays in different ways, X-ray powder diffraction (XPD) can also be used to characterize polymorphs. In addition, thermal methods such as differential scanning calorimetry (DSC) can provide unique information on a particular polymorph.

As is well known in the field of powder X-ray diffraction, relative peak heights of powder X-ray diffraction spectra can be used to describe different crystalline forms. Accordingly, in a 'Form 3' aspect, the crystalline form of compound of formula (I) displays a reflection, stated as a 2Θ value, at 17.8 ± 0.2°, in an X-ray powder diffraction pattern, wherein the reflection at 17.8 ± 0.2° is one of the four strongest reflections in the X-ray powder diffraction pattern. Preferably, the reflection at 17.8 ± 0.2° is one of the three strongest reflections in the X-ray powder diffraction pattern, or wherein the reflection at 17.8 ± 0.2° is one of the two strongest reflections in the X-ray powder diffraction pattern. More preferably, the reflection at 17.8 ± 0.2° is the strongest reflection in the X-ray powder diffraction pattern. More preferably still, in the 'Form 3' aspect, the crystalline form of compound of formula (I) displays a reflection, stated as a 2Θ value, at 17.8 ± 0.1°, in an X-ray powder diffraction pattern, wherein the reflection at 17.8 ± 0.1° is one of the four strongest reflections in the X-ray powder diffraction pattern. Preferably, the reflection at 17.8 ± 0.1° is one of the three strongest reflections in the X-ray powder diffraction pattern, or wherein the reflection at 17.8 ± 0.1° is one of the two strongest reflections in the X-ray powder diffraction pattern. More preferably, the reflection at 17.8 ± 0.1° is the strongest reflection in the X-ray powder diffraction pattern.

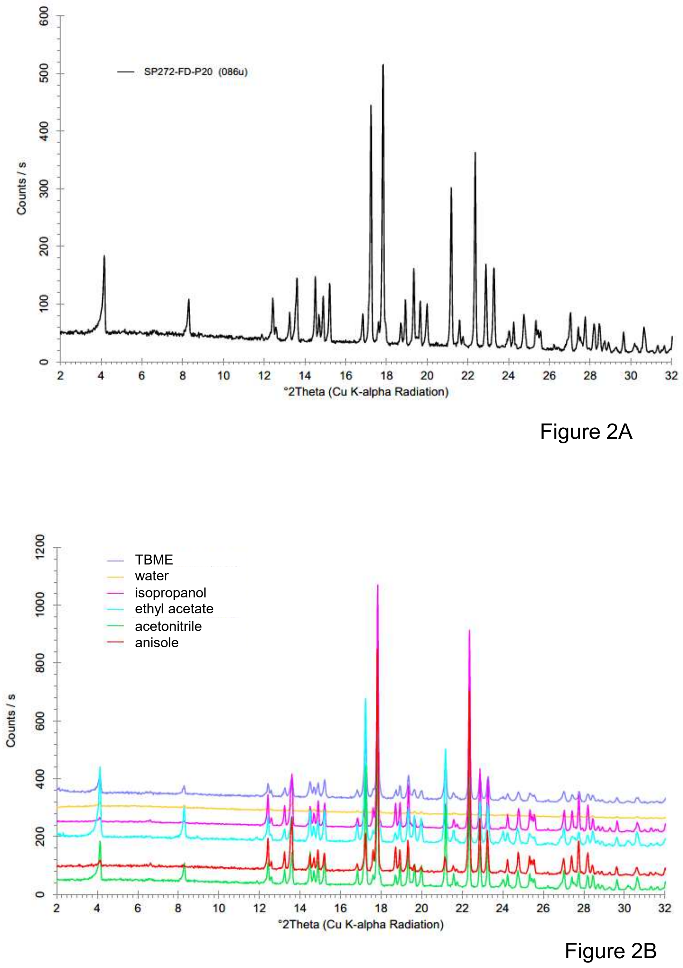

The term "strongest reflection" describes the highest peak of an X-ray powder diffraction pattern. The height of a peak of a powder X-ray diffraction pattern is determined based on the X-ray intensity (in counts or counts/sec units). Thus, the strongest reflection is a reflection that shows the highest X-ray intensity in the X-ray powder diffraction pattern. For example, the strongest reflection of the X-ray diffraction pattern shown in Figure 2A is the reflection, stated as a 2Θ value, at 17.8 ± 0.2°.

Unless explicitly stated to the contrary, all X-ray powder diffraction patterns are determined using copper K-alpha radiation at about 25 °C.

Preferably, in the 'Form 3' aspect, the crystalline form of compound of formula (I) further displays one or more reflections, stated as a 2Θ value, at one or more of 4.1 ± 0.2°, 8.3 ± 0.2°, 12.4 ± 0.2°, 13.6 ± 0.2°, 14.5 ± 0.2°, 14.9 ± 0.2°, 15.2 ± 0.2°, 17.2 ± 0.2°, 19.3 ± 0.2°, 21.2 ± 0.2°, 22.4 ± 0.2°, 22.9 ± 0.2° and 23.3 ± 0.2°, in an X-ray powder diffraction pattern. More preferably still, in the 'Form 3' aspect, the crystalline form of compound of formula (I) further displays one or more reflections, stated as a 2Θ value, at one or more of 4.1 ± 0.1°, 8.3 ± 0.1°, 12.4 ± 0.1°, 13.6 ± 0.1°, 14.5 ± 0.1°, 14.9 ± 0.1°, 15.2 ± 0.1°, 17.2 ± 0.1°, 19.3 ± 0.1°, 21.2 ± 0.1°, 22.4 ± 0.1°, 22.9 ± 0.1° and 23.3 ± 0.1°, in an X-ray powder diffraction pattern.

The positions of peaks of powder X-ray diffraction spectra are relatively insensitive to experimental details. Thus, the crystalline compounds of the invention can be characterized by a powder X-ray diffraction pattern having certain peak positions. Accordingly, in the 'Form 3' aspect, the crystalline form of compound of formula (I) is preferably characterized by reflections, stated as a 2Θ value, at 17.2 ± 0.2°, 17.8 ± 0.2°, 21.2 ± 0.2° and 22.4 ± 0.2°, in an X-ray powder diffraction pattern. More preferably, in the 'Form 3' aspect, the crystalline form of compound of formula (I) is characterized by reflections, stated as a 2Θ value, at 4.1 ± 0.2°, 8.3 ± 0.2°, 12.4 ± 0.2°, 13.6 ± 0.2°, 14.5 ± 0.2°, 14.9 ± 0.1°, 15.2 ± 0.2°, 17.2 ± 0.2°, 17.8 ± 0.2°, 19.3 ± 0.2°, 21.2 ± 0.2°, 22.4 ± 0.2°, 22.9 ± 0.2° and 23.3 ± 0.2°, in an X-ray powder diffraction pattern. More preferably still, in the 'Form 3' aspect, the crystalline form of compound of formula (I) is characterized by reflections, stated as a 2Θ value, at 17.2 ± 0.1°, 17.8 ± 0.1°, 21.2 ± 0.1° and 22.4 ± 0.1°, in an X-ray powder diffraction pattern. Most preferably, in the 'Form 3' aspect, the crystalline form of compound of formula (I) is characterized by reflections, stated as a 2Θ value, at 4.1 ± 0.1°, 8.3 ± 0.1°, 12.4 ± 0.1°, 13.6 ± 0.1°, 14.5 ± 0.1°, 14.9 ± 0.1°, 15.2 ± 0.1°, 17.2 ± 0.1°, 17.8 ± 0.1°, 19.3 ± 0.1°, 21.2 ± 0.1°, 22.4 ± 0.1°, 22.9 ± 0.1° and 23.3 ± 0.1°, in an X-ray powder diffraction pattern.

The crystalline forms of a compound can be characterized by a differential scanning calorimetry (DSC) thermogram. Thus, the crystalline form of compound of formula (I), in the 'Form 3' aspect, is preferably characterized by a DSC thermograph, which shows an onset of endothermic heat flow at about 87 °C and/or a melting point of about 92.4°C. Accordingly, preferably, in the 'Form 3' aspect, the crystalline form of compound of formula (I) has a melting point of 89 °C to 96 °C, as determined by DSC. Preferably, the crystalline form, in the 'Form 3' aspect, has a melting point of 90 °C to 95 °C, as determined by DSC. More preferably still the crystalline form of compound of formula (I), in the 'Form 3' aspect, has a melting point of 91 °C to 94 °C, as determined by DSC. Most preferably the crystalline form of compound of formula (I), in the 'Form 3' aspect, has a melting point of 92 °C to 93 °C, as determined by DSC.

A relatively high melting point (typically greater than about 80°C) of a therapeutic compound favours resistance to decomposition thereby facilitating the storage and increasing shelf life of the therapeutic compound, which is desirable for any pharmaceutical agent.

The crystalline forms of a compound can be characterized by their hygroscopicity. Hygroscopicity of a product expresses the increase or decrease in its water content as a function of relative humidity at a certain temperature. Substantially non-hygroscopic products exhibit no or only a slight change in their water content as a consequence of variations in relative humidity. In strongly hygroscopic products, water content may vary widely. Accordingly, preferably the crystalline form of compound of formula (I) is substantially non-hygroscopic. When preparing pharmaceutical compositions and formulations for use in such tablets, it is highly desirable to have a crystalline form of the therapeutic compound having low levels of hygroscopicity and/or low levels of deliquescence thereby allowing the compound to be compressed into a desired shape or size.

Substantially non-hygroscopic substances show a water absorption of less than about 2 % at a relative humidity of about 95% measured at a temperature of about 25 °C. Preferably, water absorption is less than about 1 % at a relative humidity of about 95% measured at a temperature of about 25 °C. The values of water absorption are obtained by measuring the mass gain of the tested crystalline form at a relative humidity of about 95% and a temperature of about 25 °C relative to the initial mass.

Accordingly, the crystalline form of compound of formula (I) preferably absorbs 0 % to 2 % water at a relative humidity of about 95% at a temperature of about 25 °C. More preferably still the crystalline form of compound of formula (I) absorbs 0 % to 1.5 % water at a relative humidity of about 95% at a temperature of about 25 °C. Most preferably the crystalline form of compound of formula (I) absorbs 0 % to 1 % water at a relative humidity of about 95% at a temperature of about 25 °C.

In a 'Form 2' aspect, the crystalline form of compound of formula (I) displays a reflection, stated as a 2Θ value, at 16.9 ± 0.2°, in an X-ray powder diffraction pattern, wherein the reflection at 16.9 ± 0.2° is one of the four strongest reflections in the X-ray powder diffraction pattern. Preferably, the reflection at 16.9 ± 0.2° is one of the three strongest reflections in the X-ray powder diffraction pattern, or wherein the reflection at 16.9 ± 0.2° is one of the two strongest reflections in the X-ray powder diffraction pattern. More preferably, the reflection at 16.9 ± 0.2° is the strongest reflection in the X-ray powder diffraction pattern. More preferably still, the crystalline form, in the 'Form 2' aspect, displays a reflection, stated as a 2Θ value, at 16.9 ± 0.1°, in an X-ray powder diffraction pattern, wherein the reflection at 16.9 ± 0.1° is one of the four strongest reflections in the X-ray powder diffraction pattern. Preferably, the reflection at 16.9 ± 0.1° is one of the three strongest reflections in the X-ray powder diffraction pattern, or wherein the reflection at 16.9 ± 0.1° is one of the two strongest reflections in the X-ray powder diffraction pattern. More preferably, the reflection at 16.9 ± 0.1° is the strongest reflection in the X-ray powder diffraction pattern.

Preferably, the crystalline form of compound of formula (I), in the 'Form 2' aspect, displays one or more reflections, stated as a 2Θ value, at one or more of 15.2 ± 0.2°, 16.1 ± 0.2°, 16.5 ± 0.2°, 18.9 ± 0.2°, 23.1 ± 0.2°, 25.5 ± 0.2°, 27.7 ± 0.2° and 28.5 ± 0.2°, in an X-ray powder diffraction pattern. More preferably still, in the 'Form 2' aspect, the crystalline form of compound of formula (I) displays one or more reflections, stated as a 2Θ value, at one or more of 15.2 ± 0.1°, 16.1 ± 0.1°, 16.5 ± 0.1°, 18.9 ± 0.1°, 23.1 ± 0.1°, 25.5 ± 0.1°, 27.7 ± 0.1° and 28.5 ± 0.1°.

This crystalline form of compound of formula (I), in the 'Form 2' aspect, can also be characterized by reflections, stated as a 2Θ value, at 16.1 ± 0.2°, 16.5 ± 0.2°, 16.9 ± 0.2°, 18.9 ± 0.2° and 23.1 ± 0.2°, in an X-ray powder diffraction pattern. Preferably, the crystalline form of compound of formula (I), in the 'Form 2' aspect, can also be characterized by reflections, stated as a 2Θ value, at 16.1 ± 0.1°, 16.5 ± 0.1°, 16.9 ± 0.1°, 18.9 ± 0.1° and 23.1 ± 0.1°, in an X-ray powder diffraction pattern.

The crystalline form of compound of formula (I), in the 'Form 2' aspect, can be characterized by a DSC thermograph which shows an onset of endothermic heat flow at about 58 °C and a melting point of about 70 °C. Accordingly, the crystalline form of compound of formula (I), in the 'Form 2' aspect, has a melting point of 67 °C to 74 °C. Preferably, the crystalline form of compound of formula (I), in the 'Form 2' aspect, has a melting point of 68 °C to 73 °C. More preferably still the crystalline form of compound of formula (I), in the 'Form 2' aspect, has a melting point of 69 °C to 72 °C. Most preferably the crystalline form of compound of formula (I), in the 'Form 2' aspect, has a melting point of 69 °C to 71 °C.

Preferably, the crystalline form of compound of formula (I), in the 'Form 2' aspect, is particularly soluble in water. Accordingly, preferably the crystalline form of compound of formula (I), in the 'Form 2' aspect, has water solubility of about 75 mg/mL to about 85 mg/mL, measured at about 25 °C. More preferably the crystalline form, in the 'Form 2' aspect, has water solubility of about 78 mg/mL to about 82 mg/mL, measured at about 25 °C. Most preferably the crystalline form of compound of formula (I), in the 'Form 2' aspect, has water solubility of about 80 mg/mL, measured at about 25 °C.

Methods for measuring solubility are known in the art; for example, shake-flask method, sonication, column elution method and ultraviolet or visible spectroscopy method. Unless explicitly stated to the contrary, water solubility is determined using the shake-flask method and/or sonication.

Increasing survival of the subject extends the life or life expectancy of the subject by at least 5%, at least 10%, at least 15%, at least 20%, at least 25%, at least 30%, at least 35%, at least 40%, at least 45%, at least 50%, at least 55%, or at least 60% as compared to the life (or life expectancy) the subject would have without treatment with the compound of formula (I). In other words, administration of the compound of formula (I) prolongs the life of the subject suffering from Sandhoff disease (or other GM2 gangliosidosis) as compared to the life the subject would have if the subject have not been administered the compound of formula (I). Administration of the compound of formula (I) may prolong life (and therefore delay death) of a subject suffering from Sandhoff disease by at least 1 month, at least 3 months, at least 6 months, at least 9 months, at least 1 year, at least 2 years, at least 3 years, at least 4 years, at least 5 years, at least 6 years, at least 7 years, at least 8 years, at least 9 years, at least 10 years, at least 11 years, at least 12 years, at least 13 years, at least 14 years, at least 15 years, at least 16 years, at least 17 years, at least 18 years, at least 19 years, at least 20 years, at least 25 years, at least 30 years, at least 35 years, at least 40 years, at least 45 years, or at least 50 years. Administration of the compound of formula (I) may prevent death resulting from Sandhoff disease (or other GM2 gangliosidosis) of a subject suffering from the disease. For example, the subject treated with the compound of formula (I) may be expected to live longer by at least 5 years as compared to their life without treatment with compound of formula (I).

In some embodiments, the therapeutically effective amount (i.e. amount effective to deliver a desired effect) of the compound of formula (I) (or the crystalline form thereof) may be in the range of about 0.0001 milligrams total to about 50 milligrams total of the compound of formula (I) (or crystalline form thereof) per day (mg total/day). In specific embodiments, the therapeutically effective amount may be at least about 0.001 mg total/day, at least about 0.05 mg total/day, at least about 0.1 mg total/day, at least about 0.25 mg total/day, at least about 0.5 mg total/day, at least about 1 mg total/day, at least about 3 mg total/day, at least about 5 mg total/day, or at least about 8 mg total/day. In further embodiments, the therapeutically active amount may be in the range of about 0.01-20 mg total/day, 0.05-15 mg total/day or 0.1-12 mg total/day.

Thus, the compound of formula (I) (or the crystalline form thereof, preferably 'Form 3') may be administered to the subject at a dosage ranging from about 0.0001 mg total/day to about 50 mg total/day. Preferably, the compound of formula (I) (or the crystalline form thereof, preferably 'Form 3') is administered to the subject at a dosage ranging from about 0.1 mg total/day to about 15 mg total/day.

The dose of the compound of formula (I) (or the crystalline form thereof, preferably 'Form 3') throughout the treatment period of the subject may remain the same or it may be increased or decreased. The exact maintenance amount, according to all aspects of the invention, can be routinely determined by the skilled person based on the specific subject's physical condition, severity of Sandhoff disease or other GM2 gangliosidosis symptoms and tolerance of the compound being administered. Generally speaking, the exact maintenance amount will be a compromise between improvement of the Sandhoff symptoms (or other GM2 gangliosidosis) against the physiological tolerance of the specific subject for that exact amount (e.g. the observance/potential for unwanted side effects). This is specific to each individual subject and it is routine for the skilled person in the art (e.g. a medical practitioner) to determine the exact maintenance amount for a specific subject based on the aforementioned factors.

In particular embodiments, the compound of formula (I) (or crystalline form thereof) may be administered as a single dose once per day. The compound of formula (I) (or crystalline form thereof) may be administered as two, three or more separate doses throughout the day. That is to say, the therapeutically effective amount per day is divided into two, three or more doses administered to the subject separately throughout the day. The multiple doses per day do not necessarily need to be the same amount.

The treatment (administration of the compound of formula (I), on a regular basis, such as daily) may be carried out for a suitable period of time, such as at least 2, 3, 4, 5, 6, 7, 8, 9 or 10 weeks, months or years. For example, the compound of formula (I) (or the crystalline form thereof) may be administered to the subject for at least 1 month, at least 3 months, at least 6 months, at least 9 months, at least 1 year, at least 2 years, at least 3 years. If it is well tolerated, the treatment may be carried out indefinitely. Alternatively, the compound of formula (I) (or the crystalline form thereof) may be administered to the subject one time.

Administration can be accomplished either by oral administration via tablets (such as dispersible tablets), pills, capsules, granules, powders, solutions, and the like, or parenteral administration, such as injections such as intra-articular, intravenous, and intramuscular injections, suppositories, ophthalmic solutions, eye ointments, or agents for external use, such as transdermal liquid preparations, ointments, transdermal patches, transmucosal liquid preparations, transmucosal patches, inhalers, and the like.

The compound of formula (I) (or the crystalline form thereof) may be administered to the subject together with an anti-inflammatory agent. In some embodiments, the compound of formula (I) (or the crystalline form thereof) and the anti-inflammatory agent are administered simultaneously. In some embodiments, the compound of formula (I) (or the crystalline form thereof) and the anti-inflammatory agent are administered separately, for example sequentially. In some embodiments, the compound of formula (I) (or the crystalline form thereof) and the anti-inflammatory agent are co-formulated as a single pharmaceutical composition. Alternatively, the compound of formula (I) (or the crystalline form thereof) and the anti-inflammatory agent may be formulated as a separate pharmaceutical composition. In both cases, pharmaceutical compositions may further comprise one or more pharmaceutically acceptable additives/solvents.

The anti-inflammatory agent may be a non-steroidal anti-inflammatory agent. The anti-inflammatory agent may be selected from aspirin, ibuprofen, naproxen, diclofenac, celecoxib, mefenamic acid, etoricoxib, and indomethacin.

Accordingly, the invention also provides a therapeutic combination comprising the compound of formula (I) (or the crystalline form thereof) and an anti-inflammatory agent.

Alternatively, or additionally, the compound of formula (I) (or the crystalline form thereof) may deliver the anti-inflammatory effect on its own. Preferably, the compound of formula (I) (or the crystalline form thereof) is an anti-inflammatory agent. Preferably, the compound of formula (I) (or the crystalline form thereof) increases the expression of anti-inflammatory genes in the brain. Preferably, the compound of formula (I) (or the crystalline form thereof) decreases the expression of pro-inflammatory genes in the brain. For example, the compound of formula (I) (or the crystalline form thereof) may decrease the expression of a ITGAX gene in the brain. For example, the compound of formula (I) (or the crystalline form thereof) may decrease the expression of a TREM2 gene in the brain. For example, the compound of formula (I) (or the crystalline form thereof) may decrease the expression of a CXCL10 gene in the brain. Preferably, the compound of formula (I) (or the crystalline form thereof) decreases the expression of pro-inflammatory genes in the midsection of the brain. Preferably, the compound of formula (I) (or the crystalline form thereof) increases the expression of anti-inflammatory genes in the midsection of the brain.

Integrin alpha-X/beta-2 (expressed by the ITGAX gene) is a receptor for fibrinogen. It recognizes the sequence G-P-R in fibrinogen. It mediates cell-cell interaction during inflammatory responses. It is especially important in monocyte adhesion and chemotaxis.

Triggering Receptor Expressed On Myeloid Cells 2 (expressed by the TREM2 gene) is a proinflammatory membrane protein likely involved in chronic inflammation, acts as a receptor for amyloid beta-42, lipoprotein particles, binds phospholipid, e.g., sphingomyelin, regulates microglia activation and chemotaxis.

C-X-C Motif Chemokine Ligand 10 (expressed by the CXCL10 gene) is a proinflammatory molecule involved in chemotaxis, differentiation and activation of peripheral immune cells, regulation of cell growth, apoptosis and angiogenesis.

The compound of formula (I) (or the crystalline form thereof) may decrease the expression of other genes in the brain, for example, the compound of formula (I) (or the crystalline form thereof) may decrease the expression of a CYBB gene, a CD68 gene, a HPGDS gene, a C1QB gene, a C1QA gene, and/or a PLCB2 gene.

The compound of formula (I) (or the crystalline form thereof) may increase the expression of other genes in the brain, for example, the compound of formula (I) (or the crystalline form thereof) may increase the expression a SLC18A3 gene, a TH gene, a DDC gene, and/or a RET gene.

The subject treated or to be treated with the compound of formula (I) (or the crystalline form thereof) may exhibit one or more symptoms of Sandhoff disease or other GM2 gangliosidosis. Alternatively, the subject may be treated even if he or she does not exhibit any symptoms of Sandhoff disease or other GM2 gangliosidosis. The subject may be asymptomatic. The subject treated with the compound of formula (I) (or the crystalline form thereof) may have been diagnosed with Sandhoff disease or other GM2 gangliosidosis. The diagnosis of Sandhoff disease or other GM2 gangliosidosis may be performed based on genetic screening (e.g. to determine HEXB gene mutations). Alternatively, the subject treated or to be treated with the compound of formula (I) (or the crystalline form thereof) might not have been diagnosed with Sandhoff disease or other GM2 gangliosidosis.

The one or more symptoms of Sandhoff disease may include muscle weakness, decreased movement, loss of motor skills, increased reaction to loud noises, seizures, vision loss, hearing loss, intellectual disability, an eye abnormality, enlarge organs, bone abnormalities, speech difficulties, loss of cognitive function, loss of muscle coordination, and/or psychiatric problems.

These symptoms are also present in subjects with other GM2 gangliosidoses (e.g. Tay-Sachs disease).

The treatment with the compound of formula (I) (or the crystalline form thereof) may increase survival of a subject suffering from Sandhoff disease or other GM2 gangliosidosis (e.g. Tay-Sachs disease). The expression "increase survival" in this context means extending life expectancy of a subject suffering from Sandhoff disease or other GM2 gangliosidosis (e.g. Tay-Sachs disease) and receiving the treatment in comparison to the subject suffering from Sandhoff disease or other GM2 gangliosidosis (e.g. Tay-Sachs disease) and not receiving the treatment. By "increases survival" is meant an improvement in survival time compared to the situation in which the compound was not administered. They survive for significantly longer than if untreated. Thus, the compound of formula (I) may be used to increase survival time of a subject (or life expectancy of a subject) by at least 5%, at least 10%, at least 15%, at least 20%, at least 25%, at least 30%, at least 35%, at least 40%, at least 45%, at least 50%, at least 55%, or at least 60% as compared to the survival time the subject would have without treatment with compound of formula (I). In other words, the compound of formula (I) may be used to increase survival time of a subject by at least 1 month, at least 3 months, at least 6 months, at least 9 months, at least 1 year, at least 2 years, at least 3 years, at least 4 years, at least 5 years, at least 6 years, at least 7 years, at least 8 years, at least 9 years, at least 10 years, at least 11 years, at least 12 years, at least 13 years, at least 14 years, at least 15 years, at least 16 years, at least 17 years, at least 18 years, at least 19 years, at least 20 years, at least 25 years, at least 30 years, at least 35 years, at least 40 years, at least 45 years, or at least 50 years as compared to the survival time the subject would have without treatment with compound of formula (I).

The subject treated or to be treated with the compound of formula (I) (or the crystalline form thereof) may have at least one mutation in a *HEXB* gene. The at least one mutation in the *HEXB* gene preferably results in an abnormal or decreased activity of beta-hexosaminidase A and/or beta-hexosaminidase B enzymes.

The subject treated or to be treated with the compound of formula (I) (or the crystalline form thereof) may have at least one mutation in a *HEXA* gene. The at least one mutation in the *HEXA* gene preferably results in an abnormal or decreased activity of beta-hexosaminidase A enzyme.

The subject treated or to be treated with the compound of formula (I) (or the crystalline form thereof) may exhibit abnormal activity of beta-hexosaminidase A and/or beta-hexosaminidase B as compared to a healthy subject (i.e. a subject not suffering from Sandhoff disease or other GM2 gangliosidosis (e.g. Tay-Sachs disease)). The subject may exhibit decreased activity of beta-hexosaminidase A and/or beta-hexosaminidase B as compared to a healthy subject (i.e. a subject not suffering from Sandhoff disease or other GM2 gangliosidosis (e.g. Tay-Sachs disease)). The level of beta-hexosaminidase A and/or beta-hexosaminidase enzymes can be measured in a sample from the subject using standard biochemical approaches.

The compound of formula (I) (or the crystalline form thereof) may provide at least one further therapeutic benefit to the subject in addition to increasing survival of the subject (or extending life expectancy of the subject), for example, the at least one further therapeutic benefit may be selected from improved motor function, improved behaviour, reduced anxiety, improved physical condition, improved balance, reduction of seizures, improved articulation of speech, improved gait, improved swallowing and improved intellectual ability.

According to all aspects of the invention, the subject is a mammal, preferably a human. In particular embodiments, the subject is between 28 days and 30 years of age. The subject may be an infant (i.e. between 28 days and 12 months of age). The subject may be a toddler (i.e. between 12 months and 24 months of age). The subject may be a child (i.e. between 24 months and 12 years of age). The subject may be an adolescent (i.e. between 12 years and 17 years of age). The subject may be a young adult (i.e. between 17 years and 30 years of age). The invention is, however, also applied to adults above 30 years of age in some embodiments.

The compound of formula (I) (or the crystalline form thereof) may be administered to the subject as a pharmaceutical composition. The pharmaceutical composition may have a pH value of between 2.0 and 8.0, between 3.0 and 7.0, between 4.0 and 7.5, between 4.0 and 6.0, or between 4.5 and 5.5. Preferably, the pharmaceutical composition has a pH value of between 4.5 and 5.5. The pharmaceutical composition may further comprise an acid and/or a base. For example, the pharmaceutical composition may comprise an acid to lower the pH to a desired pH value. The base may be used to increase the pH to a desired pH value. The skilled person would easily be able to determine suitable acids and bases to manipulate the pH value of the pharmaceutical composition. For example, the acid may be citric acid, acetic acid, or hydrochloric acid. For example, the base may be sodium hydroxide or potassium hydroxide.

The pharmaceutical compositions of the invention are typically prepared by pharmaceutically acceptable carrier and one or more optional ingredients. If necessary or desired, the resulting uniformly blended mixture can then be shaped or loaded into tablets (such as dispersible tablets), capsules, pills, canisters, cartridges, dispensers, and the like using conventional procedures and equipment.

When intended for oral administration in a solid dosage form (i.e., as capsules, tablets (such as dispersible tablets), pills and the like), the pharmaceutical compositions of the invention will typically comprise the compound of formula (I) (or the crystalline form thereof) as the active ingredient. The pharmaceutical composition of the invention may comprise the compound of formula (I) (or the crystalline form thereof) and no other ingredient. The pharmaceutical composition of the invention may be contained in a capsule. The pharmaceutical composition of the invention may be contained in the capsule without any other ingredient. The capsule may be a gelatine capsule or a hydroxypropyl methylcellulose (HPMC) capsule. Alternatively, the pharmaceutical composition of the invention may comprise the compound of formula (I) (or the crystalline form thereof) as the active ingredient and one or more pharmaceutically acceptable carriers. Suitable pharmaceutically acceptable carriers would be known by the person skilled in the art, for example, fats, water, physiological saline, alcohol (e.g., ethanol), glycerol, polyols, aqueous glucose solution, extending agent, disintegrating agent, binder, lubricant, wetting agent, stabilizer, emulsifier, dispersant, preservative, sweetener, colorant, seasoning agent or aromatizer, concentrating agent, diluent, buffer substance, solvent or solubilizing agent, chemical for achieving storage effect, salt for modifying osmotic pressure, coating agent or antioxidant, saccharides such as lactose or glucose; starch of corn, wheat or rice; fatty acids such as stearic acid; inorganic salts such as magnesium metasilicate aluminate or anhydrous calcium phosphate; synthetic polymers such as polyvinylpyrrolidone or polyalkylene glycol; alcohols such as stearyl alcohol or benzyl alcohol; synthetic cellulose derivatives such as methylcellulose, carboxymethylcellulose, ethylcellulose or hydroxypropylmethylcellulose, agents regulating the pH of the composition, such as bases and/or acids; and other conventionally used additives such as gelatin, talc, plant oil and gum arabic.

The oral dosage form may further be a capsule delayed release, in which the compound of formula (I) (or the crystalline form thereof) is enclosed within either a hard or soft soluble container made from a suitable form of gelatin or hydroxypropyl methylcellulose (HPMC), and which releases which the compound of formula (I) (or the crystalline form thereof) at a time other than promptly after administration, whereby enteric-coated articles are delayed release dosage forms. Capsule delayed release pellets, in which the compound of formula (I) (or the crystalline form thereof) is enclosed within either a hard or soft container or "shell" are also useful. In these cases, the compound of formula (I) (or the crystalline form thereof) itself is in the form of granules to which enteric coating has been applied, thus delaying release of the agent until its passing into the intestine. Capsule extended release and capsule film-coated extended release are also useful.

Additionally, the capsule may be covered in a designated film coating which releases the compound of formula (I) (or the crystalline form thereof) in such a manner to allow at least a reduction in dosing frequency as compared to that presented as a conventional dosage form. Examples include capsule gelatin coated (a solid dosage form in which the compound of formula (I) (or the crystalline form thereof) is enclosed within either a hard or soft soluble container made from a suitable form of gelatin or HPMC; through a banding process, the capsule is coated with additional layers of gelatin or HPMC so as to form a complete seal) and capsule liquid filled (a solid dosage form in which the compound of formula (I) (or the crystalline form thereof) is enclosed within a soluble, gelatin shell which is plasticized by the addition of a polyol, such as sorbitol or glycerin, and is therefore of a somewhat thicker consistency than that of a hard shell capsule).

In some embodiments, the compound of formula (I) (or the crystalline form thereof) may be dissolved or suspended in a liquid vehicle or formulated as a granule (a small particle or grain), a pellet (a small sterile solid mass consisting of a highly purified compound of formula (I) (or crystalline form thereof), with or without excipients, made by the formation of granules, or by compression and moulding), or a pellet coated extended release (a solid dosage form in which the compound of formula (I) (or the crystalline form thereof) itself is in the form of granules to which varying amounts of coating have been applied, and which releases the compound of formula (I) (or the crystalline form thereof) in such a manner to allow a reduction in dosing frequency as compared to that presented as a conventional dosage form).

Other forms include pills (a small, round solid dosage form containing the compound of formula (I) (or the crystalline form thereof) intended for oral administration), powder (an intimate mixture of dry, finely divided compound of formula (I) (or the crystalline form thereof) with one or more pharmaceutically acceptable additives that may be intended for internal or external use), elixir (a clear, pleasantly flavoured, sweetened hydroalcoholic liquid containing dissolved compound of formula (I) (or the crystalline form thereof); it is intended for oral use), chewing gum (a sweetened and flavoured insoluble plastic material of various shapes which when chewed, releases the compound of formula (I) (or the crystalline form thereof) into the oral cavity), syrup (an oral solution containing the compound of formula (I) (or the crystalline form thereof) and high concentrations of sucrose or other sugars; the term has also been used to include any other liquid dosage form prepared in a sweet and viscid vehicle, including oral suspensions), tablet (a solid dosage form containing the compound of formula (I) (or the crystalline form thereof) with or without suitable diluents), tablet chewable (a solid dosage form containing the compound of formula (I) (or the crystalline form thereof) with or without suitable diluents that is intended to be chewed, producing a pleasant tasting residue in the oral cavity that is easily swallowed and does not leave a bitter or unpleasant after-taste), tablet coated or tablet delayed release, dispersible tablet, tablet effervescent, tablet extended release, tablet film coated, or tablet film coated extended release where the tablet is formulated in such manner as to make the contained compound of formula (I) (or the crystalline form thereof) available over an extended period of time following ingestion. For example, the compound of formula (I) (or the crystalline form thereof) may be administered to a subject in the form of a dispersible tablet.

In other forms, a tablet for solution, tablet for suspension, tablet multilayer, tablet multilayer extended release may be provided, where the tablet is formulated in such manner as to allow at least a reduction in dosing frequency as compared to that presented as a conventional dosage form. A tablet orally disintegrating, tablet orally disintegrating delayed release, tablet soluble, tablet sugar coated, osmotic, and the like are also suitable.

As used herein, injectable and infusion dosage forms include, but are not limited to, a liposomal injectable, which either consists of or forms liposomes (a lipid bilayer vesicle usually composed of phospholipids which is used to encapsulate the compound of formula (I) (or the crystalline form thereof)); an injection, which includes a sterile preparation intended for parenteral use; an emulsion injection, which includes an emulsion consisting of a sterile, pyrogen-free preparation intended to be administered parenterally; or a lipid complex injection.

Other forms include a powder for solution injection, which is a sterile preparation intended for reconstitution to form a solution for parenteral use; a powder for suspension injection that is a sterile preparation intended for reconstitution to form a suspension for parenteral use; a powder lyophilized for liposomal suspension injection, which is a sterile freeze dried preparation intended for reconstitution for parenteral use which has been formulated in a manner that would allow liposomes (a lipid bilayer vesicle usually composed of phospholipids which is used to encapsulate the compound of formula (I) (or the crystalline form thereof), either within a lipid bilayer or in an aqueous space) to be formed upon reconstitution; or a powder lyophilized for solution injection, wherein lyophilization ("freeze drying") is a process which involves the removal of water from products in the frozen state at extremely low pressures.

As used herein, a "solution injection" refers to a liquid preparation containing the compound of formula (I) (or the crystalline form thereof) dissolved in a suitable solvent or mixture of mutually miscible solvents that is suitable for injection. A "solution concentrate injection" refers to a sterile preparation for parenteral use which, upon the addition of suitable solvents, yields a solution conforming in all respects to the requirements for injections.

A suspension injection comprises a liquid preparation, suitable for injection, which consists of solid particles dispersed throughout a liquid phase in which the particles are not soluble that can also consist of an oil phase dispersed throughout an aqueous phase, or vice-versa. A suspension liposomal injection comprises a liquid preparation, suitable for injection, which consists of an oil phase dispersed throughout an aqueous phase in such a manner that liposomes (a lipid bilayer vesicle usually composed of phospholipids which is used to encapsulate the compound of formula (I) (or the crystalline form thereof), either within a lipid bilayer or in an aqueous space) are formed. A suspension sonicated injection comprises a liquid preparation, suitable for injection, which consists of solid particles dispersed throughout a liquid phase in which the particles are not soluble. In addition, the product is sonicated while a gas is bubbled through the suspension, and this results in the formation of microspheres by the solid particles.

The parenteral carrier system includes one or more pharmaceutically suitable excipients, such as solvents and co-solvents, solubilizing agents, wetting agents, suspending agents, thickening agents, emulsifying agents, chelating agents, buffers, pH adjusters, antioxidants, reducing agents, antimicrobial preservatives, bulking agents, protectants, tonicity adjusters, and special additives. Formulations suitable for parenteral administration conveniently comprise a sterile oily or aqueous preparation of the compound of formula (I) (or the crystalline form thereof) which is preferably isotonic with the blood of the recipient, but this is not essential.

As used herein, inhalation dosage forms include, but are not limited to, an aerosol (a product that is packaged under pressure and contains the compound of formula (I) (or the crystalline form thereof) which is released upon activation of an appropriate valve system intended for topical application to the skin as well as local application into the nose (nasal aerosols), mouth (lingual and sublingual aerosols), or lungs (inhalation aerosols)). A foam aerosol is a dosage form containing the compound of formula (I) (or the crystalline form thereof), surfactants, aqueous or nonaqueous liquids, and propellants, whereby if the propellant is in the internal (discontinuous) phase (i.e., of the oil-in-water type), a stable foam is discharged, and if the propellant is in the external (continuous) phase (i.e., of the water-in-oil type), a spray or a quick-breaking foam is discharged. A metered aerosol is a pressurized dosage form consisting of metered dose valves which allow for the delivery of a uniform quantity of spray upon each activation. A powder aerosol is a product that is packaged under pressure and contains the compound of formula (I) (or the crystalline form thereof), in the form of a powder, that is released upon activation of an appropriate valve system. An aerosol spray is an aerosol product which utilizes a compressed gas as the propellant to provide the force necessary to expel the product as a wet spray and being applicable to solutions of the compound of formula (I) (or the crystalline form thereof) in aqueous solvent(s).

As discussed briefly above, the pharmaceutical composition comprising the crystalline forms of compound (I) can also be administered transdermally or transmucosally using known delivery systems and excipients. For example, the pharmaceutical composition can be admixed with permeation enhancers such as propylene glycol, polyethylene glycol monolaurate, azacycloalkan-2-ones and the like, and incorporated into a patch or similar delivery system. Additional excipients including gelling agents, emulsifiers, and buffers, may also be used. As used herein, transdermal dosage form includes, but is not limited to, a patch (a drug delivery system that often contains an adhesive backing that is usually applied to an external site on the body, whereby the ingredients (including the compound of formula (I) (or the crystalline form thereof)) either passively diffuse from, or are actively transported from, some portion of the patch, and whereby depending upon the patch, the ingredients (including the compound of formula (I) (or the crystalline form thereof)) are either delivered to the outer surface of the body or into the body. Various types of transdermal patches such as matrix, reservoir and others are known in the art.

As used herein, the topical dosage form includes various dosage forms known in the art such as lotions (an emulsion, liquid dosage form, whereby this dosage form is generally for external application to the skin), lotion augmented (a lotion dosage form that enhances delivery of the compound of formula (I) (or the crystalline form thereof), whereby augmentation does not refer to the strength of the compound of formula (I) (or the crystalline form thereof) in the dosage form), gels (a semisolid dosage form that contains a gelling agent to provide stiffness to a solution or a colloidal dispersion, whereby the gel may contain suspended particles) and ointments (a semisolid dosage form, usually containing less than 20% water and volatiles and greater than 50% hydrocarbons, waxes, or polyols as the vehicle, whereby this dosage form is generally for external application to the skin or mucous membranes). Further embodiments include ointment augmented (an ointment dosage form that enhances compound delivery, whereby augmentation does not refer to the strength of the compound of formula (I) (or the crystalline form thereof) in the dosage form), creams (an emulsion, semisolid dosage form, usually containing greater than 20% water and volatiles and/or less than 50% hydrocarbons, waxes, or polyols may also be used as the vehicle, whereby this dosage form is generally for external application to the skin or mucous membranes) and cream augmented (a cream dosage form that enhances compound delivery, whereby augmentation does not refer to the strength of the compound of formula (I) (or the crystalline form thereof) in the dosage form). As used herein, an "emulsion" refers to a dosage form consisting of a two-phase system comprised of at least two immiscible liquids, one of which is dispersed as droplets, internal or dispersed phase, within the other liquid, external or continuous phase, generally stabilized with one or more emulsifying agents, whereby emulsion is used as a dosage form term unless a more specific term is applicable (e.g. cream, lotion, ointment). Further embodiments include suspensions (a liquid dosage form that contains solid particles dispersed in a liquid vehicle), suspension extended release, pastes (a semisolid dosage form, containing a large proportion, 20-50%, of solids finely dispersed in a fatty vehicle, whereby this dosage form is generally for external application to the skin or mucous membranes), solutions (a clear, homogeneous liquid dosage form that contains one or more chemical substances dissolved in a solvent or mixture of mutually miscible solvents), and powders.

The topical dosage form composition contains the compound of formula (I) (or the crystalline form thereof) and one or more inactive pharmaceutical ingredients such as excipients, colorants, pigments, additives, fillers, emollients, surfactants (e.g., anionic, cationic, amphoteric and nonionic), penetration enhancers (e.g., alcohols, fatty alcohols, fatty acids, fatty acid esters and polyols), and the like. Various FDA-approved topical inactive ingredients are found at the FDA's "The Inactive Ingredients Database" that contains inactive ingredients specifically intended as such by the manufacturer.

Preferably the pharmaceutical composition comprises one or more further pharmaceutically active agents (e.g. anti-inflammatory agents, such as Aspirin). This combination therapy involves using a crystalline form of compound (I) combined with one or more of the further pharmaceutically active agents, either formulated together (for example, packaged together in a single formulation) or formulated separately (for example, packaged as separate unit dosage forms).

The methods and uses described herein may be *in vitro* methods (or uses) or *in vivo* methods (or uses).

The above methods and uses may be combined with a method of diagnosis of GM2 gangliosidosis (e.g. Sandhoff disease or Tay Sachs disease, preferably Sandhoff disease), in which GM2 gangliosidosis (e.g. Sandhoff disease or Tay Sachs disease, preferably Sandhoff disease) is diagnosed first in a subject, followed by administration of the compound of formula (I) (or the crystalline form thereof) to deliver a desired therapeutic effect (e.g. increasing survival) to the subject suffering from GM2 gangliosidosis (e.g. Sandhoff disease or Tay Sachs disease, preferably Sandhoff disease). The method of diagnosis may comprise genetic screening of HEXA and/or HEXB gene to determine the presence of at least one mutation. The method of diagnosis may comprise determining the levels of beta-hexosaminidase A and/or beta-hexosaminidase B in a subject. At least one mutation in HEXA gene may be indicative of Tay Sachs disease. At least one mutation in HEXB gene may be indicative of Sandhoff disease.

The foregoing detailed description has been provided by way of explanation and illustration, and is not intended to limit the scope of the appended claims. Many variations in the presently preferred embodiments illustrated herein will be apparent to one of ordinary skill in the art, and remain within the scope of the appended claims and their equivalents.

The invention is further disclosed in the following clauses:
1. A compound of formula (I), 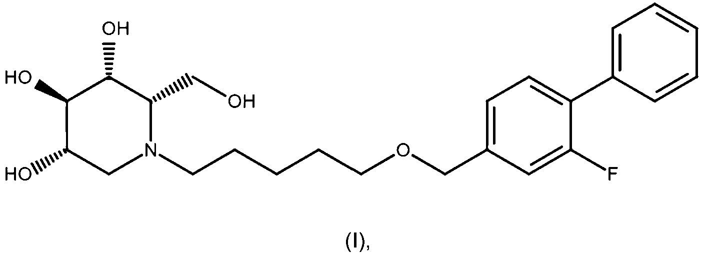 for use in improving motor function of a subject.
2. The compound for use of clause 1, wherein the subject suffers from a disease involving abnormal levels of glucosylceramide and/or higher levels of glycosphingolipids.
3. The compound for use of clause 1 or clause 2, wherein the subject suffers from GM2 gangliosidosis, in particular Sandhoff disease or Tay-Sachs disease, preferably Sandhoff disease.
4. A compound of formula (I), 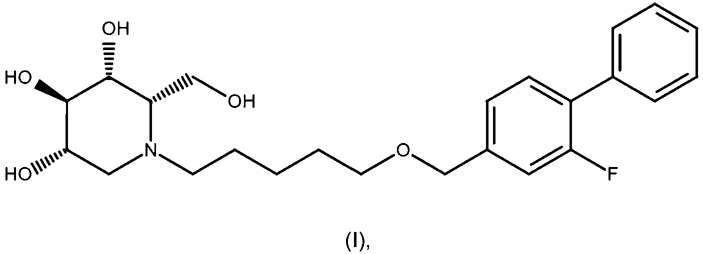 for use in treating GM2 gangliosidosis, in particular Sandhoff disease or Tay-Sachs disease, preferably Sandhoff disease.
5. A compound of formula (I), 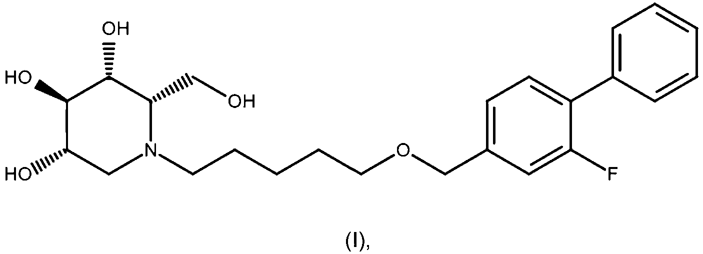 for use in alleviating symptoms of GM2 gangliosidosis, in particular Sandhoff disease or Tay-Sachs disease, preferably Sandhoff disease.
6. A compound of formula (I), 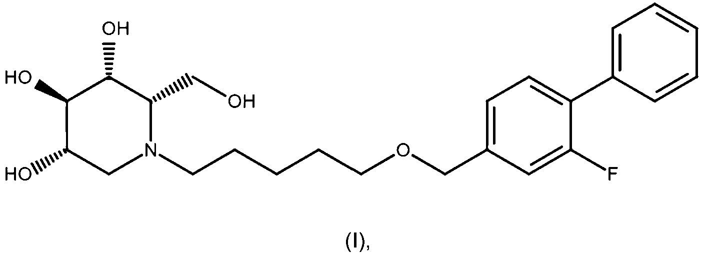 for use in extending life of a subject suffering from GM2 gangliosidosis, in particular Sandhoff disease or Tay-Sachs disease, preferably Sandhoff disease.
7. A compound of formula (I), 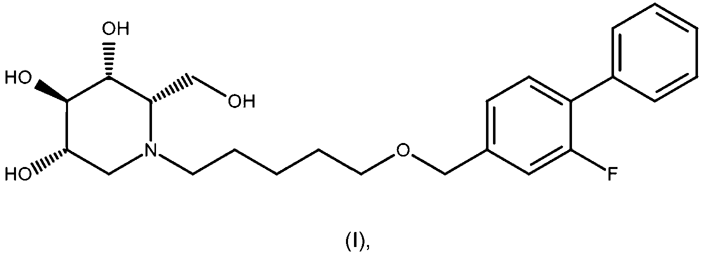 for use in increasing survival of a subject suffering from GM2 gangliosidosis, in particular Sandhoff disease or Tay-Sachs disease, preferably Sandhoff disease.
8. The compound for use of any one of clauses 1-7, wherein the compound is in a crystalline form.
9. The compound for use of clause 8, wherein the crystalline form is a crystalline free base.
10. The compound for use of clause 8 or clause 9, wherein the crystalline form displays a reflection, stated as a 2Θ value, at 17.8 ± 0.2°, in an X-ray powder diffraction pattern, wherein the reflection at 17.8 ± 0.2° is one of the four strongest reflections in the X-ray powder diffraction pattern.
11. The compound for use of clause 10, wherein the crystalline form further displays one or more reflections, stated as a 2Θ value, at one or more of 4.1 ± 0.2°, 8.3 ± 0.2°, 12.4 ± 0.2°, 13.6 ± 0.2°, 14.5 ± 0.2°, 14.9 ± 0.2°, 15.2 ± 0.2°, 17.2 ± 0.2°, 19.3 ± 0.2°, 21.2 ± 0.2°, 22.4 ± 0.2°, 22.9 ± 0.2° and 23.3 ± 0.2°, in an X-ray powder diffraction pattern.
12. The compound for use of clause 8 or clause 9, wherein the crystalline form displays reflections, stated as a 2Θ value, at 17.2 ± 0.2°, 17.8 ± 0.2°, 21.2 ± 0.2° and 22.4 ± 0.2°, in an X-ray powder diffraction pattern.
13. The compound for use of any one of clauses 8-12, wherein the crystalline form has a melting point of 89°C to 96°C.
14. The compound for use of any one of clauses 8-13, wherein the crystalline form has a melting point of 91°C to 94°C.
15. The compound for use of any one of clauses 8-14, wherein the crystalline form has a melting point of 92°C to 93°C.
16. The compound for use of any one of clauses 8-15, wherein the crystalline form is substantially non-hygroscopic.
17. The compound for use of clause 8 or clause 9, wherein the crystalline form displays a reflection, stated as a 2Θ value, at 16.9 ± 0.2°, in an X-ray powder diffraction pattern, wherein the reflection at 16.9 ± 0.2° is one of the four strongest reflections in the X-ray powder diffraction pattern.
18. The compound for use of clause 17, wherein the crystalline form further displays one or more reflections, stated as a 2Θ value, at one or more of 15.2 ± 0.2°, 16.1 ± 0.2°, 16.5 ± 0.2°, 18.9 ± 0.2°, 23.1 ± 0.2°, 25.5 ± 0.2°, 27.7 ± 0.2° and 28.5 ± 0.2°, in an X-ray powder diffraction pattern.
19. The compound for use of any one of clauses 7-18, wherein increasing survival extends the life expectancy of the subject by at least 5%, at least 10%, at least 15%, at least 20%, at least 25%, at least 30%, at least 35%, or at least 40%.
20. The compound for use of any one of clauses 7-19, wherein increasing survival extends life expectancy of a subject who has been administered the compound in comparison to a subject who has not been administered the compound.
21. The compound for use of any one of clauses 1-20, wherein the compound is administered to the subject at a dosage ranging from about 0.0001 mg total/day to about 50 mg total/day.
22. The compound for use of any one of clauses 1-21, wherein the compound is administered to the subject at a dosage ranging from about 0.1 mg total/day to about 15 mg total/day.
23. The compound for use of any one of clauses 1-22, wherein the compound is administered to the subject together with an anti-inflammatory agent.
24. The compound for use of clause 23, wherein the compound is administered to the subject simultaneously or sequentially with the anti-inflammatory agent.
25. The compound for use of clause 23 or clause 24, wherein the anti-inflammatory agent is aspirin, ibuprofen, naproxen, diclofenac, celecoxib, mefenamic acid, etoricoxib, or indomethacin.
26. The compound for use of any one of clauses 1-25, wherein the compound is administered to the subject daily.
27. The compound for use of any one of clauses 1-26, wherein the compound is administered to the subject at least 1, at least 2, at least 3 times a day.
28. The compound for use of any one of clauses 1-27, wherein the subject exhibits one or more symptoms of GM2 gangliosidosis, in particular Sandhoff disease or Tay-Sachs disease, preferably Sandhoff disease.
29. The compound for use of clause 28, wherein the one or more symptoms of GM2 gangliosidosis, in particular Sandhoff disease or Tay-Sachs disease, preferably Sandhoff disease include: muscle weakness, decreased movement, loss of motor skills, increased reaction to loud noises, seizures, vision loss, hearing loss, intellectual disability, an eye abnormality, enlarge organs, bone abnormalities, speech difficulties, loss of cognitive function, loss of muscle coordination, and/or psychiatric problems.
30. The compound for use of any one of clauses 1-27, wherein the subject is asymptomatic.
31. The compound for use of any one of clauses 1-27, wherein the subject does not exhibit any symptoms of GM2 gangliosidosis, in particular Sandhoff disease or Tay-Sachs disease, preferably Sandhoff disease.
32. The compound for use of any one of clauses 1-31, wherein the subject has been diagnosed with GM2 gangliosidosis, in particular Sandhoff disease or Tay-Sachs disease, preferably Sandhoff disease.
33. The compound for use of any one of clauses 28-32, wherein the compound increases survival of a subject suffering from GM2 gangliosidosis, in particular Sandhoff disease or Tay-Sachs disease, preferably Sandhoff disease, and wherein increasing survival extends life expectancy of a subject suffering from GM2 gangliosidosis, in particular Sandhoff disease or Tay-Sachs disease, preferably Sandhoff disease in comparison to a subject not suffering from GM2 gangliosidosis, in particular Sandhoff disease or Tay-Sachs disease, preferably Sandhoff disease.
34. The compound for use of any one of clauses 1-31, wherein the subject has not been diagnosed with GM2 gangliosidosis, in particular Sandhoff disease or Tay-Sachs disease, preferably Sandhoff disease.
35. The compound for use of any one of clauses 1-34, wherein the subject has at least one mutation in a *HEXB* gene.
36. The compound for use of clause 35, wherein the compound increases survival of a subject suffering from GM2 gangliosidosis, in particular Sandhoff disease or Tay-Sachs disease, preferably Sandhoff disease, and wherein increasing survival extends life expectancy of a subject having at least one mutation in a *HEXB* gene in comparison to a subject not having any mutations in the *HEXB* gene.
37. The compound for use of any one of clauses 1-36, wherein the subject exhibits abnormal activity of beta-hexosaminidase A and/or beta-hexosaminidase B.
38. The compound for use of any one of clauses 1-37, wherein the subject exhibits decreased activity of beta-hexosaminidase A and/or beta-hexosaminidase B as compared to a healthy subject.
39. The compound for use of clause 37 or clause 38, wherein the compound increases survival of a subject suffering from GM2 gangliosidosis, in particular Sandhoff disease or Tay-Sachs disease, preferably Sandhoff disease, and wherein increasing survival extends life expectancy of a subject having abnormal or decreased activity of beta-hexosaminidase A and/or beta-hexosaminidase B in comparison to a subject having a normal activity of beta-hexosaminidase A and/or beta-hexosaminidase B.
40. The compound for use of any one of clauses 1-39, wherein the compound provides at least one further therapeutic benefit to the subject, wherein the at least one further therapeutic benefit is selected from improved motor function, improved behaviour, reduced anxiety, improved physical condition, improved balance, reduction of seizures, improved articulation of speech, improved gait, improved swallowing and improved intellectual ability.
41. The compound for use of any one of clauses 1-40, wherein the subject is human.
42. The compound for use of clause 41, wherein the subject is between 28 days and 30 years of age.
43. The compound for use of any one of clauses 1-42, wherein the compound is administered to the subject as a pharmaceutical composition which has a pH of between 4.0 and 7.5.
44. The compound for use of clause 43, wherein the pharmaceutical composition has a pH of between 4.5 and 5.5.
45. The compound for use of clause 43 or 44, wherein the pharmaceutical composition further comprises an acid and/or a base.
46. The compound for use of any one of clause 43-45, wherein the pharmaceutical composition is administered to the subject in a capsule or a tablet, such as a dispersible tablet.
47. The compound for use of any one of clauses 1-46 wherein the compound is administered to the subject for at least 1 month, at least 3 months, at least 6 months, at least 9 months, at least 1 year, at least 2 years, at least 3 years.
48. The compound for use of any one of clauses 1-46 wherein the compound is administered to the subject one time.
49. A method of increasing survival of a subject suffering from GM2 gangliosidosis, in particular Sandhoff disease or Tay-Sachs disease, preferably Sandhoff disease, the method comprising administering to the subject suffering from GM2 gangliosidosis, in particular Sandhoff disease or Tay-Sachs disease, preferably Sandhoff disease a compound of formula (I) 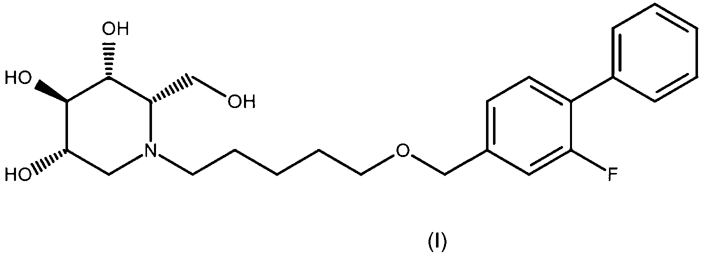 in an amount effective to increase survival of the subject.
50. A method of treating a subject suffering from GM2 gangliosidosis, in particular Sandhoff disease or Tay-Sachs disease, preferably Sandhoff disease, wherein the method increases survival of the subject, the method comprising administering to the subject suffering from GM2 gangliosidosis, in particular Sandhoff disease or Tay-Sachs disease, preferably Sandhoff disease a compound of formula (I) 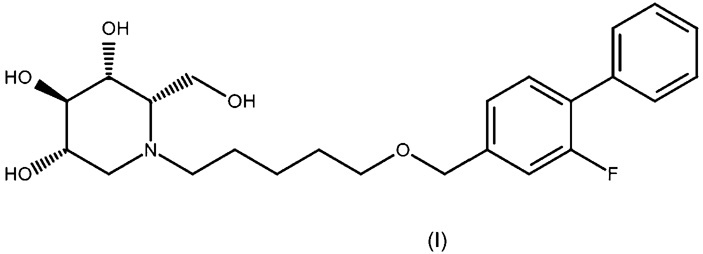 in an amount effective to increase survival of the subject.
51. Use of a compound of formula (I) 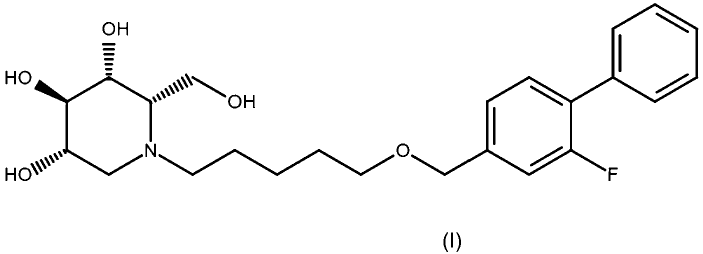 for increasing survival of a subject suffering from GM2 gangliosidosis, in particular Sandhoff disease or Tay-Sachs disease, preferably Sandhoff disease.

The invention is further disclosed in the following further clauses:
1. A compound of formula (I), 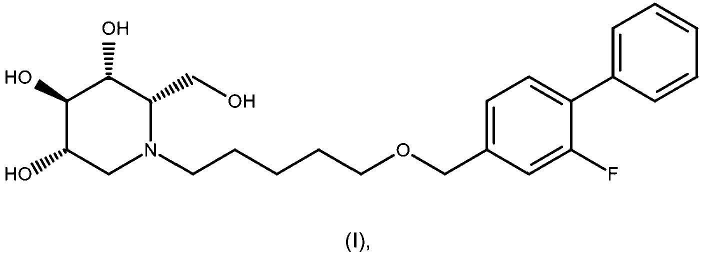 for use in increasing survival of a subject suffering from GM2 gangliosidosis, in particular Sandhoff disease or Tay-Sachs disease, preferably Sandhoff disease.
2. A compound of formula (I), 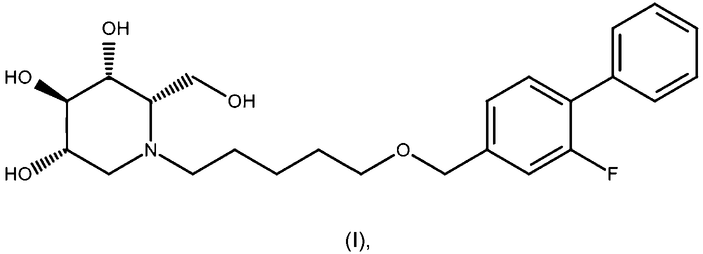 for use as an anti-inflammatory agent, preferably in a subject suffering from a GM2 gangliosidosis.
3. The compound for use of clause 1 or 2, wherein the compound is in a crystalline form.
4. The compound for use of clause 3, wherein the crystalline form is a crystalline free base.
5. The compound for use of clause 3 or clause 4, wherein the crystalline form displays a reflection, stated as a 2Θ value, at 17.8 ± 0.2°, in an X-ray powder diffraction pattern, wherein the reflection at 17.8 ± 0.2° is one of the four strongest reflections in the X-ray powder diffraction pattern.
6. The compound for use of clause 5, further displaying one or more reflections, stated as a 2Θ value, at one or more of 4.1 ± 0.2°, 8.3 ± 0.2°, 12.4 ± 0.2°, 13.6 ± 0.2°, 14.5 ± 0.2°, 14.9 ± 0.2°, 15.2 ± 0.2°, 17.2 ± 0.2°, 19.3 ± 0.2°, 21.2 ± 0.2°, 22.4 ± 0.2°, 22.9 ± 0.2° and 23.3 ± 0.2°, in an X-ray powder diffraction pattern.
7. The compound for use of any one of clauses 1 and 3-5, wherein increasing survival extends the life expectancy of the subject by at least 5%, at least 10%, at least 15%, at least 20%, at least 25%, at least 30%, at least 35%, or at least 40%.
8. The compound for use of any one of clauses 1-7, wherein the compound is administered to the subject at a dosage ranging from about 0.1 mg total/day to about 15 mg total/day.
9. The compound for use of any one of clauses 1-8 wherein the compound is administered to the subject together with an anti-inflammatory agent.
10. The compound for use of clause 9, wherein the compound is administered to the subject simultaneously or sequentially with the anti-inflammatory agent.
11. The compound for use of any one of clauses 1-10, wherein the compound is administered to the subject daily.
12. The compound for use of any one of clauses 1-11, wherein the subject:
   (a) exhibits one or more symptoms of GM2 gangliosidosis, in particular Sandhoff disease or Tay-Sachs disease, preferably Sandhoff disease;
   (b) does not exhibit any symptoms of GM2 gangliosidosis, in particular Sandhoff disease or Tay-Sachs disease, preferably Sandhoff disease;
   (c) has been diagnosed with GM2 gangliosidosis, in particular Sandhoff disease or Tay-Sachs disease, preferably Sandhoff disease; or
   (d) has not been diagnosed with GM2 gangliosidosis, in particular Sandhoff disease or Tay-Sachs disease, preferably Sandhoff disease.
13. The compound for use of any one of clauses 1-12, wherein the subject has at least one mutation in a *HEXB* gene and/or shows decreased activity of beta-hexosaminidase A and/or beta-hexosaminidase B.
14. The compound for use of any one of clauses 1-13, wherein the compound provides at least one further therapeutic benefit to the subject, wherein the at least one further therapeutic benefit is selected from improved motor function, improved behaviour, reduced anxiety, improved physical condition, improved balance, reduction of seizures, improved articulation of speech, improved gait, improved swallowing and improved intellectual ability.
15. The compound for use of any one of clauses 1-14, wherein the subject is human, preferably the subject is between 28 days and 30 years of age.
16. The compound for use of any one of clauses 1-15, wherein the compound is administered to the subject in a pharmaceutical composition which has a pH of between 4.0 and 7.5.

### EXAMPLES

### Example 1 - production of crystalline form of compound (I)

A stable crystalline form of compound of formula (I) was obtained from suspension equilibration experiments of compound of formula (I) in various respective solvents, for instance, in acetonitrile, ethyl acetate, isopropanol, anisole, water, or TBME respectively, at room temperature (around 25 °C).

In particular, a Form 3 crystalline form of compound of formula (I) was obtained by the following experimental methods:
1) about 74 mg of compound of formula (I) was added to 2.0 ml of acetonitrile and the suspension was stirred for three days at room temperature (about 25 °C), then filtered;
2) about 74 mg of compound of formula (I) was added to 2.0 ml of anisole and the suspension was stirred for three days at room temperature (about 25 °C), then filtered;
3) about 82 mg of compound of formula (I) was added to 1.0 ml of ethyl acetate and the suspension was stirred for three days at room temperature (about 25 °C), then filtered;
4) about 82 mg of compound of formula (I) was added to 1.0 ml of isopropanol and the suspension was stirred for three days at room temperature (about 25 °C), then filtered;
5) about 45 mg of compound of formula (I) was added to 1.0 ml of water and the suspension was stirred for three days at room temperature (about 25 °C), then filtered;
6) about 100 mg of compound of formula (I) was added to 3.0 ml TBME and the suspension was stirred for three days at room temperature (about 25 °C), then filtered.

Compound of formula (I) was purified using a silica gel column prior to the addition of the solvent to remove borate esters.

The crystalline form of compound of formula (I) obtained was characterized by powder X-ray diffraction, DSC and DVS.

The PXRD pattern of the Form 3 crystalline form of compound of formula (I) obtained from suspension equilibration experiments with acetonitrile is shown in Figure 2A. An overlay of the PXRD patterns of the Form 3 crystalline form of compound of formula (I) obtained from suspension equilibration experiments with other solvents is shown in Figure 2B. This crystalline form of compound of formula (I) shows strongest reflections at a 2Θ value of 17.2 ± 0.2°, 17.8 ± 0.2°, 21.2 ± 0.2° and 22.4 ± 0.2°. This is consistent despite different solvents being used to obtain the crystalline form of compound of formula (I).

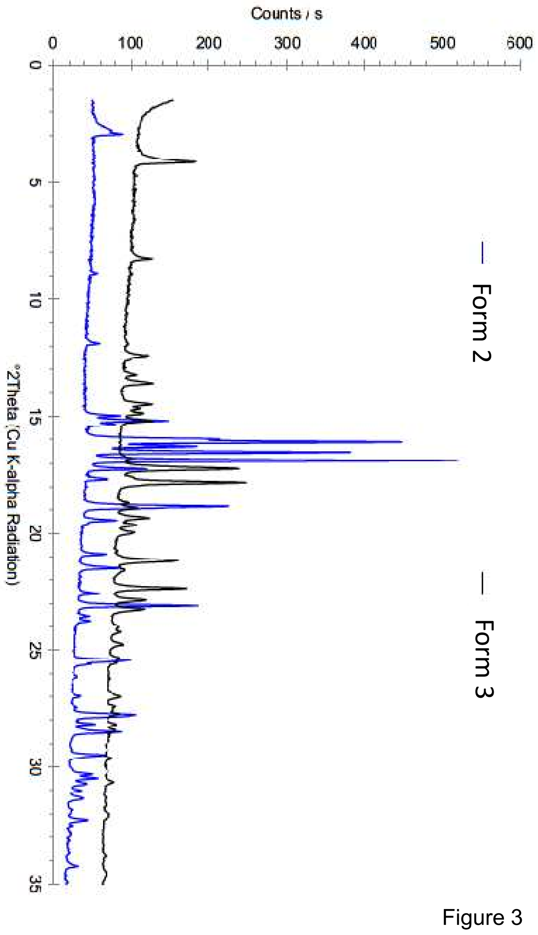

For comparison, Figure 3 shows an overlay of the PXRD pattern of another crystalline form (Form 2) of compound of formula (I) and Form 3. The two crystalline forms have a different PXRD pattern. Form 2 was obtained by a short (about 1-5 min) equilibration of purified compound of formula (I) (compound of formula (I) was purified using a silica gel column to remove borate esters) in acetonitrile. This Form 2 crystalline form of compound of formula (I) shows strongest reflections at a 2Θ value of 16.1 ± 0.2°, 16.5 ± 0.2°, 16.9 ± 0.2°, 18.9 ± 0.2° and 23.1 ± 0.2°.

Powder X-ray diffraction was carried out with a Stoe Stadi P diffractometer equipped with a Mythen1K detector operating with Cu- Kα₁ radiation. The measurements with this instrument were performed in transmission at a tube voltage of 40 kV and 40 mA tube power. A curved Ge monochromator allows testing with Cu-K α₁radiation. The following parameters were set: 0.02° 2θ step size, 12 s step time, 1.5-50.5° 20 scanning range, and 1°2θ detector step (detector mode in step scan). For a typical sample preparation about 10 mg of sample was placed between two acetate foils and mounted into a Stoe transmission sample holder. The sample was rotated during the measurement. All sample preparation and measurement was done in an ambient air atmosphere (about 25 °C).

DSC measurements performed for Form 3 show a sharp endothermic melting peak at about 92°C associated with an enthalpy of about 103 J/g. DSC measurements performed for Form 2 show first strong endotherm with a peak temperature at about 70°C and an enthalpy of about 54 J/g is followed by a weaker signal at 81°C and an enthalpy of about 3 J/g.

Differential scanning calorimetry (DSC) was carried out with a TA Instruments Q2000 instrument (closed aluminum sample pan or aluminum sample pan with a pinhole in the lid, heating rate 20 K/min). The melting point is understood as the peak maximum.

In addition, the behaviour of the sample of the Form 3 crystalline form of compound of formula (I) obtained by equilibration with ethyl acetate was investigated under variable water vapour pressures (DVS measurement). At the highest relative humidity of 95% the sample absorbed about 1.2% of water that was released when the relative humidity was returned to the 50% RH. The amount of absorbed water is small, and the water sorption is reversible. Thus, this Form 3 crystalline form of compound of formula (I) is non-hygroscopic or substantially non-hygroscopic. For Form 2, at high relative humidity the sample absorbed about 18% of water; however, most of the absorbed water was released when the relative humidity was returned to the 50% RH.

Dynamic vapour sorption (DVS) measurements were performed with an SPS11-100n "Sorptions Prüfsystem" from ProUmid (formerly "Projekt Messtechnik"), August-Nagel-Str. 23, 89079 Ulm (Germany). About 5mg to 20 mg of sample was put into an aluminum sample pan. Humidity change rates of 5% per hour were used. The applied measurement program can be described as follows:
The sample was placed on an aluminium or platinum holder on top of a microbalance and allowed to equilibrate at a relative humidity (RH) of 50% before starting the pre-defined humidity programs:
(1) 2 h at 50% RH
(2) 50 → 0% RH (5%/h); 5 h at 0% RH
(3) 0 → 95% RH (5%/h); 5 h at 95% RH
(4) 95 → 0% RH (5%/h); 5 h at 0% RH
(5) 0 → 95% RH (5%/h); 5 h at 95% RH
(6) 95 → 50% RH (5%/h); 2h at 50% RH

### Example 2 - Effect of administration of the crystalline form of compound of formula (I) to mice suffering from Sandhoff disease (Hexb null mutation)

In the Example, AZ-3102 is a name for compound of formula (I) in Form 3.

### 1. Introduction

### Background and overview of Project:

The GM2 gangliosidoses are a group of degenerative/inflammatory diseases affecting the brain which include Sandhoff disease. These diseases are characterized by rapid neurological deterioration and typically death before 4 years of age since treatment success has been limited to date. They are a result of the inheritance of defects in genes responsible for encoding the enzymes required to breakdown GM2 ganglioside (a lipid). This lipid then accumulates in the body, reaching its highest levels in the brain where it is most abundant. In their severe infantile form, these diseases become evident at about 6 months of age. Juvenile- and adult-onset forms of the disease are associated with a wide range of neurological symptoms, many of which are debilitating and ultimately lethal.

GM2 ganglioside is normally degraded in a cell's lysosomes through the concerted action of the products of three genes, HEXA, HEXB, and GM2A. A defect in any one of these genes can result in a deficiency of HEXA activity towards GM2 ganglioside, which then cannot be broken down. Mutations in HEXB, encoding the beta subunit of β-hexosaminidase, result in Sandhoff disease. The incidence of Sandhoff disease is approximately 1 in 384,000 live births, but is increased in certain populations.

Mouse models of Sandhoff diseases have been created by targeted disruptions of the mouse Hexb gene. Mutations targeted to Hexb genes result in a severe neurological phenotype characterized by extensive GM2 ganglioside accumulation in the brain and spinal cord, leading to spasticity, muscle weakness, rigidity, and ultimately death by 16-17 weeks of age. These mice are used extensively as a model for studying potential therapies for the GM2 gangliosidoses.

In addition to the genetic cause of GM2 gangliosidoses, an inflammatory response (microglial activation, macrophage infiltration, oxidative damage) has been associated with these lysosomal storage diseases. Specifically, inflammation is known to result from excess GM2 storage in the brain and conversely, chronic inflammation of the brain has been implicated in the direct as well as indirect disease pathogenesis and progression in GM2 gangliosidoses. This group also demonstrated that the inflammatory cytokine TNFα, IL1β and TGFβ1 production was elevated in brains of Sandhoff mice, but not in control mice. Later studies confirmed these findings in humans affected with GM2 gangliosidoses. Finally, macrophage activation and rise in the level of an inflammatory cytokine, Macrophage inflammatory protein-1α (MIP-1α), has been implicated in pathogenesis of Sandhoff disease.

Further evidence for a role for inflammation in GM2 has been demonstrated by the use of anti-inflammatory drug, Aspirin, which have been shown to significantly slow down the progression of disease in Sandhoff mice. When aspirin was combined with substrate reduction therapy, (treatment with compounds which inhibit the synthesis of the lipids involved in GM2 gangliosidoses) synergy was found to result (11%, improvement in survival) with a maximum improvement of 73% survival.

Another therapy for GM2 gangliosidosis that has recently shown promise is the drug pyrimethamine. Treatment of GM2 gangliosidosis cells lines with pyrimethamine recently demonstrated improved enzyme activity and has led to some further success in its initial human trials. Pyremethamine is an approved drug for treating malaria, but has been shown to enhance production of hexosaminidase.

Substrate reduction therapy (SRT), in which the primary pharmacological action is to inhibit the formation of glucosylceramide (GlcCer) by inhibiting the enzyme glucosylceramide synthase (GCS), and subsequently decreased biosynthesis of more complex GSL is an additional therapeutic principle. SRT has been approved for Gaucher type 1 and Niemann Pick diseases.

Miglustat (N-butyldeoxynojirimycin) is an iminosugar, a synthetic analogue of D-glucose that primarily inhibits GbA2 (the non-lysosomal glucosylceramidase), but might also inhibit GCS at high doses due to its relatively low potency on this enzyme. Clinically, miglustat is approved for Gaucher type 1 and approved in a few countries outside the United States for Niemann-Pick type C disease.

### 2. Objectives

Survival Study & Behavioural Study was a dose-finding study with four or more doses of daily AZ-3102 vs vehicle (control), starting at 30-day postnatal, to determine its efficacy in delaying and/or reducing progressive motor dysfunctions in adulthood and in prolonging survival in homozygous mutants.

### 3. Material & methods

### 3.1. Animals subjects:

Hexb-/- mice have been obtained from Jackson Laboratories -
(strain: B6; 129S4-Hexbtm1Rlp/J). The mouse line was maintained by crossing homozygous -/males with heterozygous females (generating litters with 50% -/-and 50% +/-) under specific pathogen-free conditions. All mice were housed in individual, ventilated cages (IVCs) with 12-h light/dark cycles with food and water ad libitum. Young-adult mice of the same genetic C57BL/6 background were from Jackson Laboratories. Breeding of Hexb mutants will target needs of the "Long-term" cohorts first, followed by the "16-week" biomarker study.

### 3.2. Breeding:

Gender- and age-matched littermates from synchronized breeding (1 male -/- with 1 female +/-) were used; homozygous mutant Hexb -/- mice and +/- littermates considered in the field as phenotypic wild-type animals (mutation is autosomal recessive) were investigated in parallel. The Hexb genotype was determined by PCR of DNA isolated from ear clippings obtained at weaning using the primers 5'- ATT TTA AAA TTC AGG CCT CGA-3' (common), 5' CAT AGC GTT GGC TAC CCG TGA-3' (mutant), and 5'-CAT TCT GCA GCG GTG CAC GGC-3' (wild-type). Expected time for breeding was approximately 19 weeks.

### 3.3. Animal identification:

At weaning, ear clippings were taken to unambiguously identify each animal within the same cage.

The samples obtained this way were used for genotyping (see section 3.2).

### 3.4. Treatment:

Mice were randomly allocated to several treatment groups including a vehicle control in each cohort. Cohorts were run sequentially. Each cohort will have male and female animals in equal proportion for the Long-term cohort and 8 males and 7 females for the 16-week cohort.

For all cohorts, drug and/or vehicle (control) were administered by gastric gavage once a day, between 8.00 - 12.00 am, from the age of 30 days until euthanasia. Within any cohort, animals were preventively euthanized for reducing distress upon occurrence of immobility, tremors or seizures, poor body condition, inability to perform righting reflex, 15% weight loss, and moribundity (refer to Appendix A for body scoring chart).

**Table 1: Treatment and animal allocation.**

| S. No. | Genotype | Treatment | Age of delivery | Drug Dose (mg/kg/day) | Number in Cohort | |
|---|---|---|---|---|---|---|
| | | | | | Endpoint (Age) | |
| | | | | | 16-weeks | Long-term |
| 1 | Heterozygote (+/-) | Vehicle | 4 weeks | -- | 15 | 18 |
| 2 | Heterozygote (+/-) | *Drug* | 4 weeks | 3 | 15 | 18 |
| 3 | Sandhoff (-/-) | *Drug* | 4 weeks | 0.5 | 15 | 18 |
| 4 | Sandhoff (-/-) | *Drug* | 4 weeks | 1.5 | 15 | 18 |
| 5 | Sandhoff (-/-) | *Drug* | 4 weeks | 3 | 15 | 18 |
| 6 | Sandhoff (-/-) | *Drug* | 4 weeks | 6 | 15 | 18 |
| 7 | Sandhoff (-/-) | Vehicle (Saline) | 4 weeks | -- | 15 | 18 |
| | | | | Total | 105 | 126 |

Timed bleeding from mice occurred prior to dosing (post-natal week 4) and again 4 weeks after dosing (post-natal week 8) at 0.5 h and 1 h (see section 3.10).

Assessment included live animal assessment (for survival benefit and neuromuscular strength) includes rotarod and open field.

### 3.5. Formulation Preparation:

**Table 2. Formulation details**

| | |
|---|---|
| Concentration Range | 0.01 mg/mL - 2mg/mL |
| Vehicle Control | Purified water, 1M Citric acid, 0.1M NaOH |
| Stability | 8 days |
| Storage | 15-25°C |
| Equipment for preparation | Glass |
| Formulation Container type | Amber Glass Jars wrapped in aluminium foil |
| Sample container types | See study plan |

1. Introduction and preparation of vehicle control
1.1. Read the method prior to proceeding with laboratory work
1.2. Read Study Plan
1.3. Ensure the work-station is clean and select the correct equipment, clean glassware etc. for the study.
1.4. Formulate in ascending order of concentration.
1.5. Start with control group - measure the required volume of purified water and magnetically stir. Whilst magnetically stirring, adjust pH to 3.5-2.5 using 1M citric acid. Record volumes in raw data.
1.6. Stir for a minimum of 30 minutes. Record stir times in raw data.
1.7. Adjust the pH to 4.5-5.5 using 0.1M NaOH. Record volumes in raw data.
1.8. Stir for a minimum of 20 minutes using a magnetic stirrer. Record stir times in the raw data.
1.9. Sample at this point if required.
2. Test item formulations
2.1. Weigh the required amount of test item: e.g., For a 0.9 mg/mL target concentration in 600 mL, weigh 540 mg.
2.2. Add 70% of the final volume of purified water to the test item to obtain a thin suspension (may be difficult to see at these low concentrations): e.g., 420 mL
2.3. Add not more than 3 molar equivalents of citric acid, and record the pH which should be pH 3.5-2.5, e.g., 3.74 mL of 1 M citric acid solution in water. Note: The suspension should clear up as an indication for dissolution of the test item.
2.4. The formulation will be stirred constantly for 30 minutes or until all the substance is dissolved. Record stir times in raw data.
2.5. Add sodium hydroxide (NaOH 0.1 M) to adjust the pH to 4.5-5.5. Note: this amount neutralizes the excess protons from the citric acid by 60% to maintain the solution under acidic conditions: e.g., 59.8 mL NaOH.
2.6. Make-up to final Volume with purified water e.g., 116 mL.
2.7. Check and record final pH. If pH is not within the range of 4.5-5.5, contact Study Director. Additional NaOH may be added, but cannot exceed 2.5% of the total volume. If required, record volumes in raw data.
2.8. Stir for a minimum of 20 minutes using a magnetic stirrer. Record start and finish times in the raw data. Continue to magnetically stir.
2.9. Sample at this point, if required, while magnetically stirring.
2.10. Transfer the solution to final containers, via syringe, whilst magnetic all stirring and flush with nitrogen.

### 3.6. Cohorts:

Cohort 1: There were a total of 6 dose groups and 2 vehicle groups using both Hexb+/- and Hexb-/- animals. AZ-3102 was investigated at doses of 0.5, 1.5, 3.0 and 6.0 mg/kg/day. Dosing occurred daily from day 30 to day 170, unless the animal was prematurely euthanized per protocol.

Cohort 2 (16-week, Biomarker Study): There were 8 groups using both Hexb+/- and Hexb-/- animals, which were administered either vehicle control or AZ-3102 at different doses, starting at 30 days postnatal.

### 3.7 Monitoring - Pharmacodynamics:

Mice were inspected on a daily basis. A behavioural & neurological assessment score test (BNA), including subjective and objective measures of general health and body weight (BW) were performed bi-weekly, from Postnatal day 56 (= earliest time of symptom development in homozygotes) onwards daily.

Motor functions were evaluated in Cohort 2 using established methods in the mouse: accelerated rotarod performance test for sensorimotor coordination (ROT) and open-field locomotor test for spontaneous spatial motor exploration (OFT).

Behavioural testing occurred after daily gavage in a separate testing room. The animals were brought to the testing room 30-60 minutes prior to the testing procedures to allow for acclimatization.

ROT/OFT was started at 8 weeks of age, again at 12 weeks of age, and thereafter every 2 weeks until the humane endpoints were reached. Each mouse was tested a minimum of 4 times in the ROT and OFT. The exact number of tests each mouse experiences depended on the number of mice which had survived at the particular time-point.

In the ROT, all animals were on the day before the first testing, habituated to the device for 5 min using a continuous rotation speed of 4 - 40 rotations per minute (rpm). For the subsequent experiments, no prior habituation was conducted. In the actual testing procedure, the test was conducted over 5 min with an accelerating rotation of 4 - 40 rpm. Time was measured until the animals fell from the rod. Up to 5 animals could be tested simultaneously.

The OFT was conducted using a 40 x 40 cm sized apparatus in a quiet room and constant lighting conditions. Trial duration was 5 min. All trials were videotaped and later analysed using the tracking software Smart^{®} Version 3.0.05. Measures included the average locomotion speed, walked distance and time spent in outer and inner zones of the field.

### 3.8. Sacrificing mice:

At the end of the defined treatment period, mice were humanely euthanized by transcardial perfusion with Ringer solution after C02 asphyxiation. Blood samples were collected before perfusion. Before perfusion, blood samples were collected via cardiac puncture into K2EDTA tubes and stored on wet ice. Whole blood was processed to plasma by centrifugation (3000 g for 10 minutes at 5°C) within 30 min of collection and stored at -75°C.

Brain samples were collected, rinsed with saline and blotted dry. Samples were fixed in 4% PFA for immunohistochemical analyses; frozen and kept for gene expression and molecular biology analyses; and used frozen for LC-MS/MS analysis.

### 3.9. Blood Sampling

At the designated time point, animals had a terminal blood draw via the descending abdominal vena cava including baseline.

Timed sampling: All mice underwent a timed sample via a saphenous vein bleed at 0.5 hr and 1h prior to the first dose and again at 4 weeks after the dose (post-natal week 8). Plasma was collected and assayed for baseline measurements, AZ-3102 and PD biomarkers.

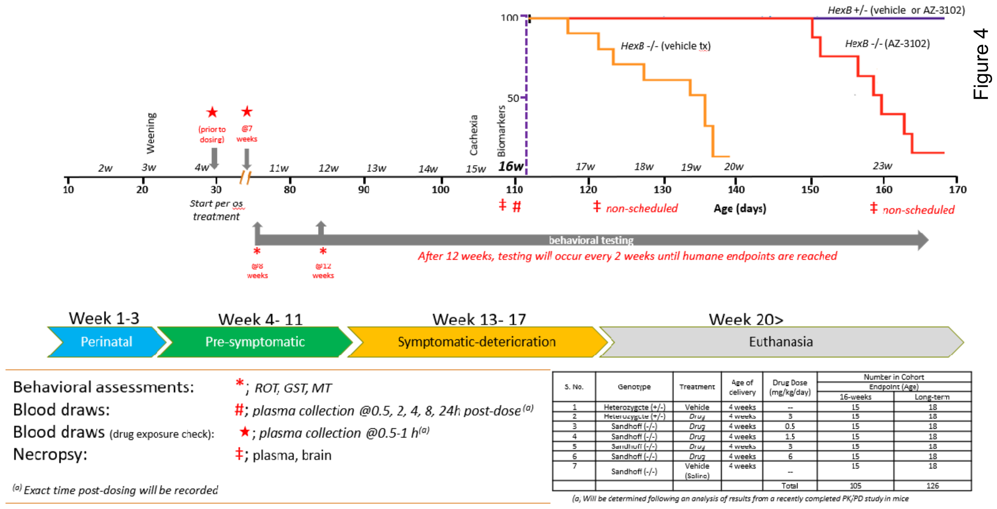

Schematic description of study protocols is shown in Figure 4.

### 4. Results

Sandhoff mouse model (Hexb (-/-)): The mouse model of Sandhoff disease is targeted to mutations in the Hexb gene that result in a severe neurological phenotype characterized by extensive GM2 ganglioside accumulation in the brain and spinal cord, leading to spasticity, muscle weakness, rigidity, and ultimately death by 16-17 weeks of age (Sango, Yamanaka et al 1995, Phaneuf, Wakamatsu et al 1996, Gulinello, Chen et al 2008). These mice are used extensively as a model for studying potential therapies for the GM2 gangliosidoses.

Hexb (-/-) mice were obtained from Jackson Laboratories - (strain: B6; 129S4-Hexbtm1Rlp/J). The mouse line was maintained by crossing homozygous (-/-) males with heterozygous females (generating litters with 50% (-/-) and 50% (+/-) under specific pathogen-free conditions. All mice were housed in individual, ventilated cages (IVCs) with 12-h light/dark cycles with food and water ad libitum. Young-adult mice of the same genetic C57BL/6 background were from Jackson Laboratories.

Mice were randomly allocated to several treatment groups including a vehicle control in each cohort (Table 3). Each cohort had male and female animals in equal proportions (N=16-18 mice). With the exception of the technician responsible for making formulations, all other staff, including the Principal Investigator and Sponsor, were blinded to the treatments.

**Table 3. Sandhoff mouse model Summary of groups and treatments**

| **Genotype** | **Treatment** | **Cohort Label** | **Age of Treatment start** | **Dose (mg/kg/day)** |
|---|---|---|---|---|
| Heterozygote (+/-) | Vehicle | HET-A | 4 weeks | -- |
| Heterozygote (+/-) | Drug | HET-B | 4 weeks | 3 |
| Sandhoff (-/-) | Vehicle | SAND-C | 4 weeks | 0 |
| Sandhoff (-/-) | Drug | SAND-E | 4 weeks | 0.5 |
| Sandhoff (-/-) | Drug | SAND-D | 4 weeks | 1.5 |
| Sandhoff (-/-) | Drug | SAND-B | 4 weeks | 3 |
| Sandhoff (-/-) | Drug | SAND-A | 4 weeks | 6 |

For all cohorts, drug and/or vehicle were administered by gastric gavage once a day, between 8.00 - 12.00 am, from the age of 30 days until euthanasia. Within any cohort, animals were preventively euthanized for reducing distress upon occurrence of immobility, tremors or seizures, poor body condition, inability to perform righting reflex, 15% weight loss, and moribundity.

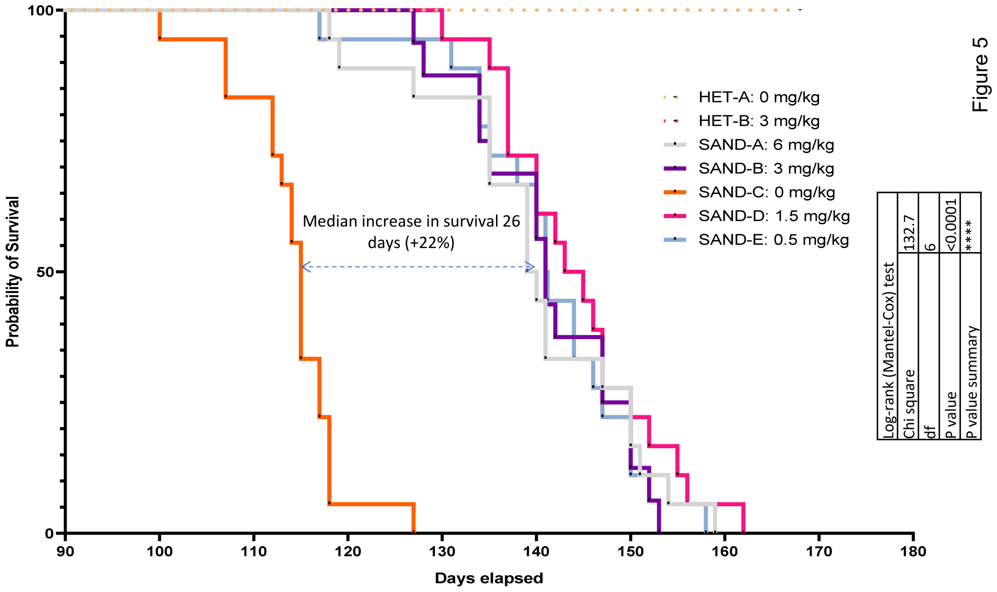

Sandhoff mice, Hexb (-/-), were treated with vehicle or AZ-3102 by oral gavage daily at dose levels of 0 (vehicle, acidified water formulation), 0.5, 1.5, 3 and 6 mg/kg/day. In addition, animals heterozygous for the Hexb gene (+/-) were treated with vehicle or 3 mg/kg/day. Dosing continued until the animals reached their humane endpoints determined in a blinded fashion by the animal care staff and primarily consisted of the inability to reach sternal recumbency after 10 seconds and, if achieved, was additionally verified by the senior laboratory technician prior to the commencement of the animal being euthanized. Median survival (n=16-18 per group) for the vehicle treated animals was 115 days. While the AZ-3102 treated animals lacked a dose-response, the average median survival was significantly increased by 26 days, a 22% increase in survival (p<0.0001, log-rank, Mantle-Cox test) (Figure 5).

Behavioral assessments, including open field tests (OFT, ActiMot, TSE systems) and rotarod (RR, IITC Life Sciences), were also performed. The OFT assessed motor skills by placing a mouse into a square box with high walls for 5 minutes to record several parameters, including distance travelled (centimetres) and resting time in zone (seconds). Motor coordination of mice was assessed using a RR apparatus as per previously published protocol (Osmon, Vyas et al. 2018). Briefly, mice were placed on moving cylinders that accelerated from 4 to 40 rotations per minute (rpm) over 5 minutes. Each mouse was allowed three trials on the RR with a minimum of 10 minutes for rest in-between assessments. The highest number achieved was recorded for each of the following parameters: latency to fall, end rotations per minute and distance travelled.

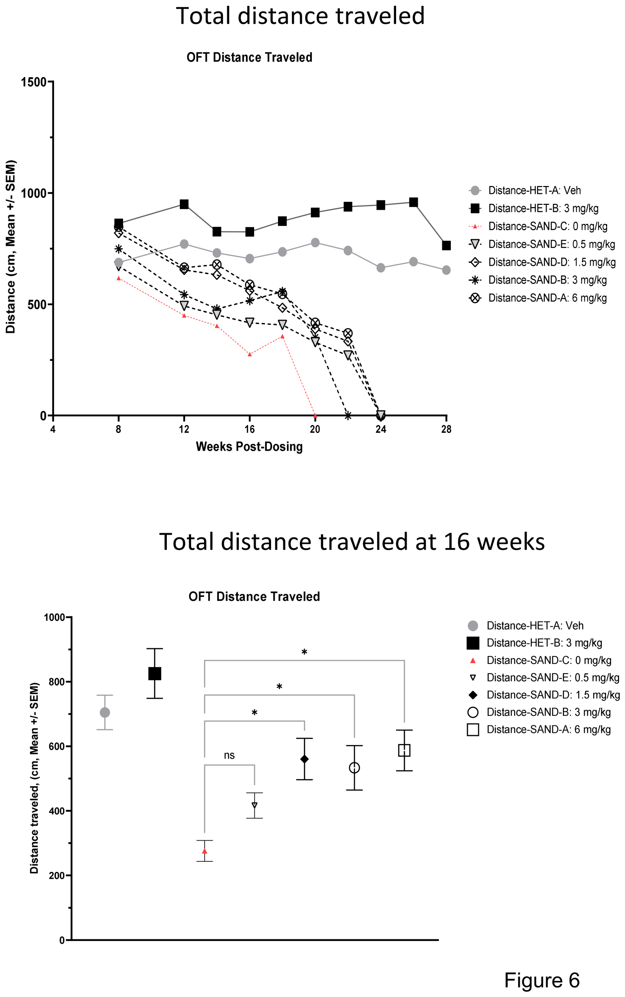

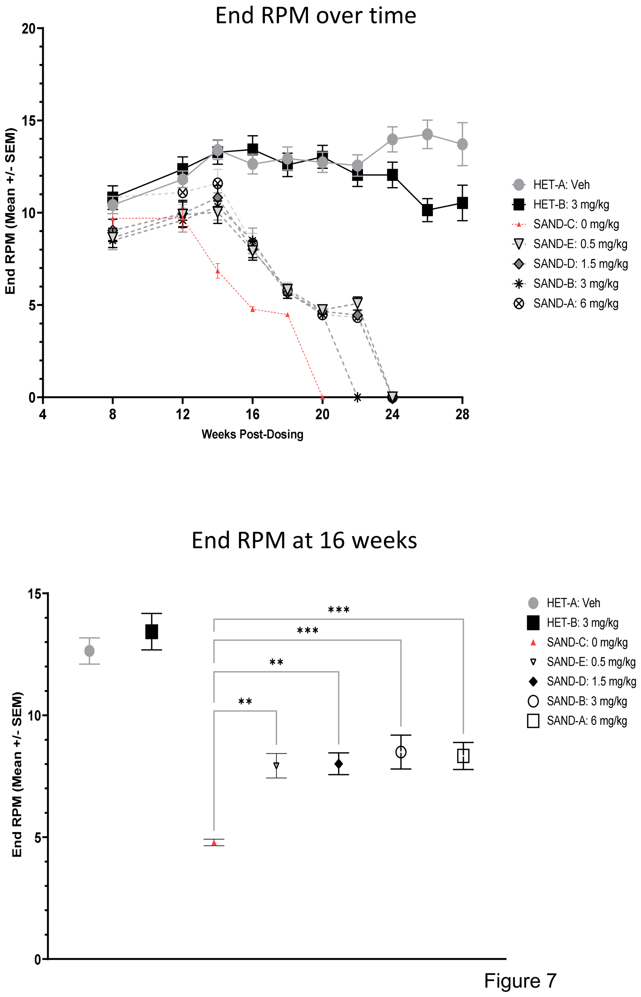

Figure 6, illustrates the performance over time in the OFT assessments for each cohort. Both Total Distance Travelled and Resting Time in Zone are improved over the course of the experiment up until the animals succumb to disease pathology. In particular, at the 16-week time point, the performance of the AZ-3102 treated Hexb (-/-) groups separate from the Hexb (-/-) vehicle treated group and, while not significant at all doses, likely demonstrate a dose-response for both measurements (Figure 6). Similarly, for the RR assessments, at 16-weeks, the effect of AZ-3102 treatment is significant at all dose levels versus vehicle treated Hexb (-/-) mice (Figure 7).

### 5. Discussion

Mouse models of Sandhoff diseases have been created by targeted disruptions of the mouse Hexb gene. Mutations targeted to Hexb genes result in a severe neurological phenotype characterized by extensive GM2 ganglioside accumulation in the brain and spinal cord, leading to spasticity, muscle weakness, rigidity, and ultimately death by 16-17 weeks of age. These mice are used extensively as a model for studying potential therapies for the GM2 gangliosidoses.

In this study, we have evaluated AZ-3102, an oral small molecule with low nM inhibitory activity on both GCS and GbA2 enzymes, in the mouse model of Sandhoff disease [Hexb (-/-)]. Sandhoff mice, Hexb (-/-), were treated with vehicle or AZ-3102 by oral gavage daily at dose levels of 0 (vehicle, acidified water formulation), 0.5, 1.5, 3 and 6 mg/kg/day. In addition, animals heterozygous for the Hexb gene (+/-) were treated with vehicle or 3 mg/kg/day. Dosing continued until the animals reached their humane endpoints determined. Median survival (n=16-18 per group) for the vehicle treated animals was 115 days. While the AZ-3102 treated animals lacked a dose-response, the average median survival was significantly increased by 26 days, a 22% increase in survival (p<0.0001, logrank, Mantle-Cox test). In addition, both the OFT and RR behavioral assessments were significantly improved with AZ-3102 treatment versus vehicle treated Hexb (-/-) animals.

These data demonstrate that AZ-3102 imparts significant survival and improved behavioral assessments in an animal model of disease that, with no treatment, would result in a significantly reduced lifespan accompanied by severe neuropathology and motor reflex deterioration. In addition, AZ-3102 administered at 3 mg/kg/day to Hexb (+/-) mice had no effect on lifespan compared to vehicle treated Hexb (+/-) animals demonstrating the relative benign effects of the drug in healthy animals.

While a dose-response relationship was not observed in the survival endpoint it is remarkable that even at 0.5 mg/kg/day dosing that the increase in survival is already present. Furthermore, while lower doses may be explored the behavioral assessments appear to indicate that 0.5 mg/kg/day may be approaching the lower limit of efficacy as the OFT results at 16-weeks indicate a potential dose-response relationship.

Future analysis with the short-term cohort will investigate pharmacokinetics of AZ-3102, glycosphingolipid modulation (e.g., GlcCer, GM2), neuropathology and gene regulation in the presence or absence of AZ-3102 treatment in Hexb (-/-) and Hexb (+/-) mice.

### Appendix A: Hexb Shaky Mice Body Scoring Chart:

Untreated Hexb mice timeline: WT and Hexb +/- do not become shaky and have a normal life span. KO mice will start to show physical symptoms around 10-12 weeks of age, severe stiffness and/or shakiness happens around 14-16 weeks of age, at which point they would be euthanized. Most untreated KO mice were either used for study control mice or as breeders.

The scoring system used to determine whether the animal should be euthanized is shown in Table 4.

**Table 4. Scoring system used to determine whether the animal should be euthanized.**

| **Numerical Score** | **Exhibited Symptoms** |
|---|---|
| 1 | • First signs of stiffness; or |
| | • Slight tremor |
| | • BCS 3 |
| 2 | • Beginning to lose weight |
| | • Stiff |
| | • BCS 2 or 3 |
| 3 | • Stiff and shaky |
| | • Steady weight loss |
| | • BCS 2 or 3 |
| 4 | • Stiff and shaky but still able to right themselves |
| | • Stiff hind limbs, slow walking |
| | • Uncoordinated |
| | • Lost weight, but has not reached their 15% weight loss |
| | • BCS 2 |
| 5 | • 15% weight loss **and/or** inability to right itself |
| | • Very restricted movement of hind limbs, "paddle- like" gait, very uncoordinated |
| | • BCS 1 or 2 |

### Example 3 - Pharmacokinetics (PK) & Pharmacodynamics (PD)

Unless otherwise stated, the methodology and sample collection were performed as in Example 2.

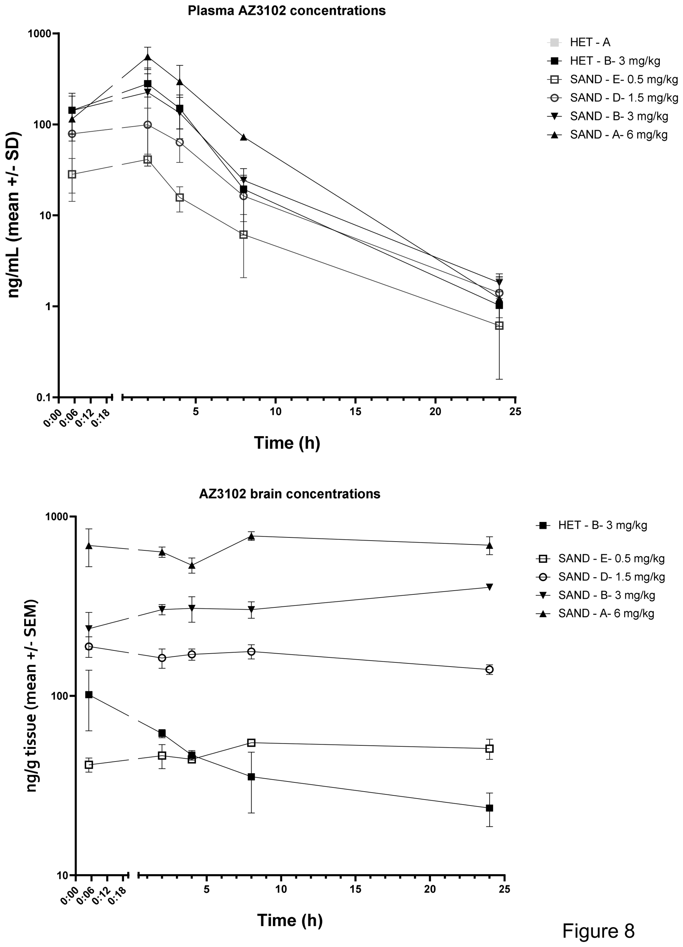

Plasma and brain PK were determined following steady-state conditions after 16-weeks administration of AZ-3102-00 (the free base of AZ-3102, which will be referred to as AZ-3102 throughout this document unless otherwise state). Twenty-four hours after the last dose the animals were euthanized at varying time-points and AZ-3102 was measured by LC- MS/MS. Over a dose range from 0.5 to 6 mg/kg/day the plasma concentration profiles showed good separation and a dose-response. The concentrations for the mean AUC₀₋₂₄ (N=3/time point) ranged from 417- 3607 ng·h/mL, and were slightly under proportional (9-fold) based on the 12-fold expected exposure ratio (Figure 8 and Table 5). Cₘₐₓ, was also under proportional with a range from 100-674 ng/mL, an approximately 7-fold difference between the lowest and highest dose. However, exposure (AUC₀₋₂₄) proportionality was well conserved between doses: 6 versus 3 (1.8-fold); 3 and 1.5 (2.0-fold) and 1.5 and 0.5 (2.4-fold). While the plasma concentration decreased with time, the concentration profile in brain was sustained with very little decrease over the 24 h period in most dose groups. The reduced clearance in the brain versus plasma has also been observed in prior studies with rats and dogs (internal data). Importantly, at all dose levels the concentrations in brain and plasma over the 24 h period was well above the IC₅₀ for the AZ-3102 for both enzyme targets. The plasma concentration at all doses was approximately 1 ng/mL after 24 h and in the brain the concentration was greater than 20 ng/g tissue. *In vitro* potency based on IC₅₀ for GCS is 1.1 ng/mL and 0.3 ng/mL for GbA2, thus the concentrations across all doses were sufficient to significantly inhibit these enzymes.

**Table 5. Pharmacokinetic Summary of AZ-3102 in the Sandhoff Model following 16-weeks of once daily administration. ^{a} A measurement of 0.8 ng/mL was found at a single timepoint and was likely due to sample contamination. ^{b} Composite following the last dose. Sampling based on the arithmetic means of 3 animals per time point.**

| **Dose (mg/kg/day)** | **Group** | **AUC₀₋₂₄ (ng·h/mL)^{b}** | **Cmax (ng/mL)^{b}** | **AUC/D (ng·h/mL)/(mg/kg)** | **Cₘₐₓ/D (ng/mL)/(mg/kg)** |
|---|---|---|---|---|---|
| 0 | Het A | - | _^{a} | - | - |
| 3 | Het B | 1958 | 370 | 653 | 123 |
| 0 | Sand C | - | - | - | - |
| 0.5 | Sand E | 417 | 100 | 834 | 200 |
| 1.5 | Sand D | 987 | 159 | 658 | 106 |
| 3 | Sand B | 1978 | 446 | 659 | 149 |
| 6 | Sand A | 3607 | 674 | 601 | 112 |

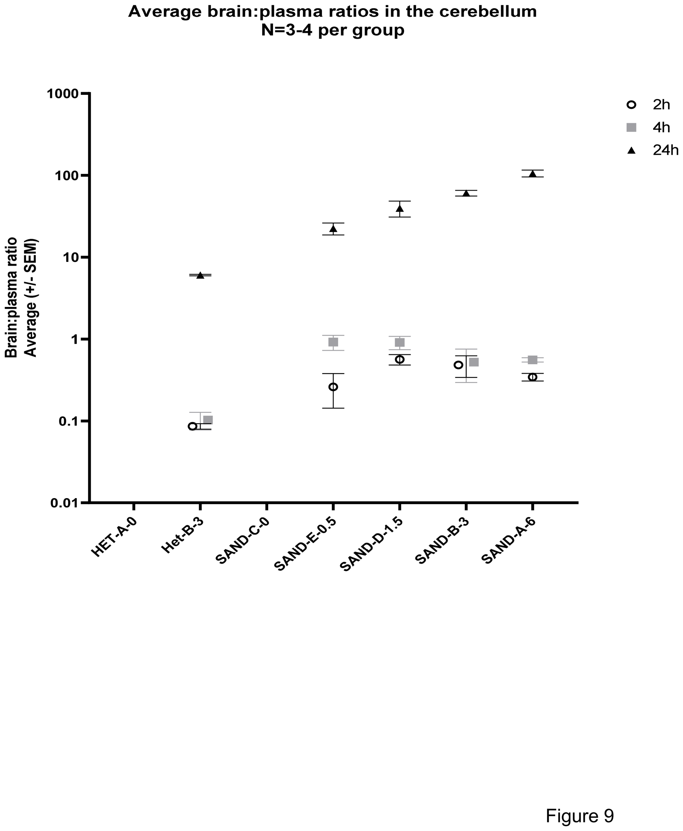

While the plasma concentration had a typical decrease with time, the concentration profile in brain was relatively sustained with very little decrease over the 24 h period in most dose groups with the exception of the NPC heterozygous animals (NPC -/+). This is highlighted in Figure 9 in which the average brain to plasma ratios are plotted at 2, 4, and 24h. In general, at 2 and 4h the ratios of AZ-3102 are greater in the Sandhoff animals compared to the heterozygous animals. In addition, while at the early time points the ratios appear to be constant, at 24 h there is an increased ratio with the increase in dose.

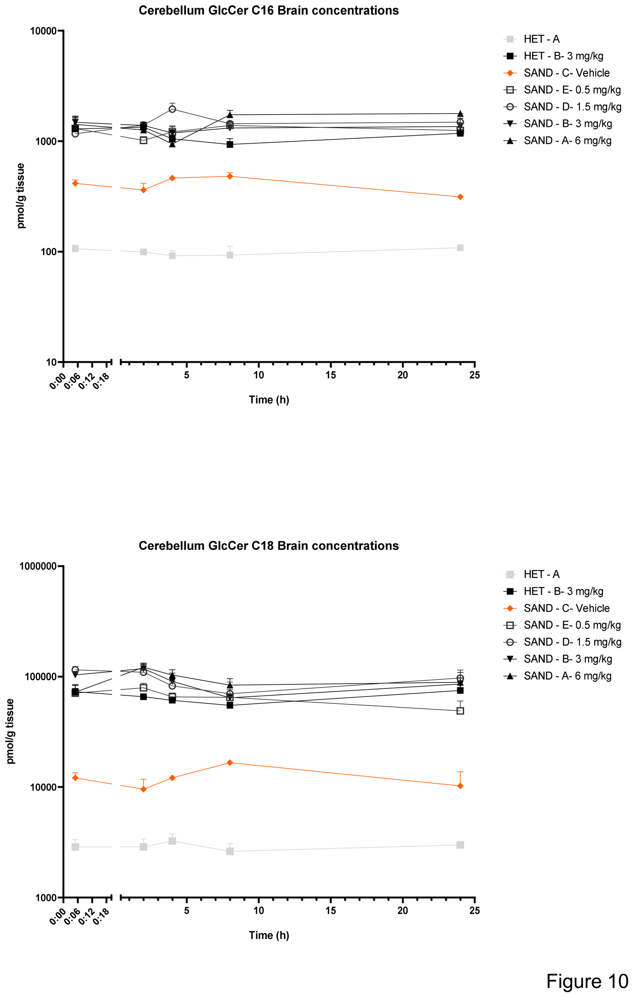

AZ-3102 is a potent inhibitor of both GCS and GbA2. Consistent with this action, the inhibition of GbA2 should result in an increase in GlcCer species in the brain where GbA2 plays a significant role. As a measurement of target engagement GlcCer C16:0 and C18:0 have been measured in the cerebellum and, as expected, there is an approximately 10-fold increase in GlcCer C16:0 and C18:0 in mice treated with AZ-3102 compared to either untreated (Heterozygous mice) or Hexb (-/-) untreated mice (Figure 10).

### Example 4 - Further survival study

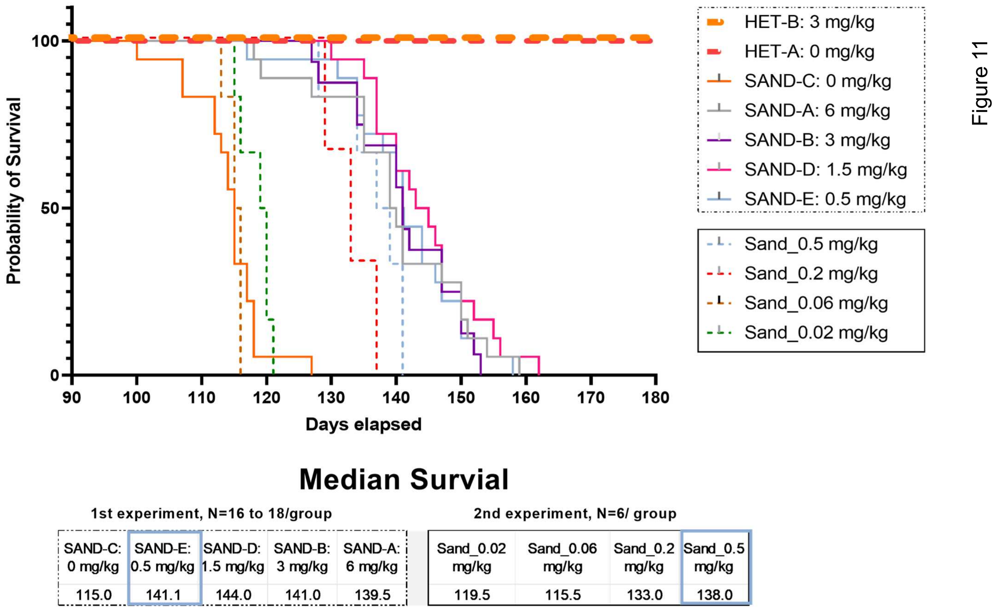

A subsequent study to explore lower doses was also performed (in the same manner as in Example 2) in which doses of 0.02, 0.06, 0.2 and 0.5 mg/kg/day were used. The two highest doses were not significantly different from each other in terms of survival, but each was significantly different from the dose of 0.02 mg/kg/day. The median survival for the Hexb (-/-) mice at 0.02, 0.06, 0.2 and 0.5 mg/kg/day was, 120, 116, 133, and 138 days. It is unclear why the 0.06 mg/kg/day had less survival as formulations were checked and confirmed accurate dosing (internal data). Nevertheless, the data confirm the repeatability of the model at the 0.5 mg/kg/day dose and demonstrate a dose response or perhaps a threshold effect below 0.02 mg/kg/day (Figure 11, taken together with data from Figure 5; Example 2).

With this dosage regimen, the 0.2 mg/kg/day dose appears to be the minimal effective dose on survival. However, it is remarkable that even at 0.5 mg/kg/day the increase in survival is already present. Furthermore, while the lower doses help to discriminate the effect on survival, the dose of 0.5 mg/kg/day appears to be approaching the lower limit of efficacy as the OFT results at 16-weeks indicate a potential dose-response relationship.

Together, these data demonstrate that AZ-3102 imparts significant survival and improved behavioral assessments in an animal model of disease that, with no treatment, would result in a significantly reduced lifespan accompanied by severe neuropathology and motor reflex deterioration. In addition, AZ-3102 administered at 3 mg/kg/day to Hexb (+/-) mice had no effect on lifespan compared to vehicle treated Hexb (+/-) animals demonstrating the benign effects of the drug in healthy animals.

### Example 5 - Gene expression

Unless otherwise stated, the methodology and sample collection were performed as in Example 2.

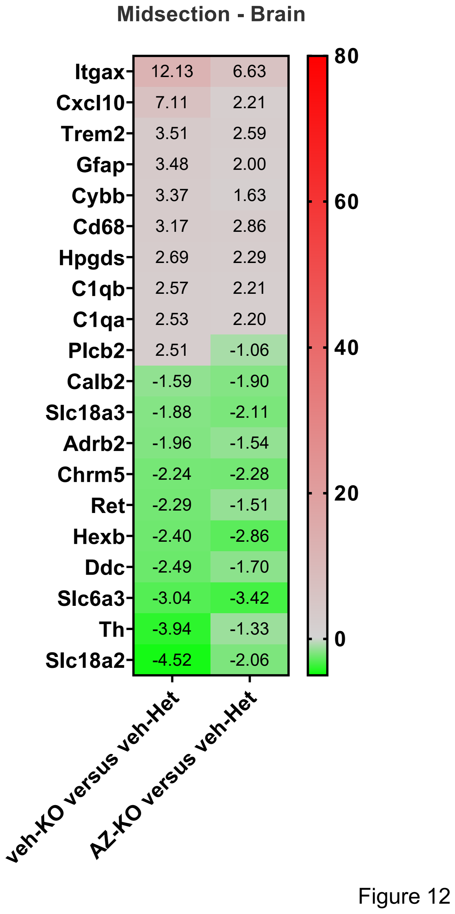

A heat map of the top 10 upregulated and downregulated genes in the midsection brain region is provided in Figure 12. While data are currently being investigated for additional insights, the most consistent observation is the effect of AZ-3102 on proinflammatory gene expression. While not exhaustive, and by means of illustration, three genes of interest in the midsection are highlighted: *Itgax* (Gene ID: 16411, integrin alpha-X, encoding CD11c surface protein) has been associated with activation of microglia, is a marker for dendritic cells and has been associated with neuropathologies, including Alzheimer's disease (Haage, V., Semtner, M., Vidal, R. O. et al. (2019) Comprehensive gene expression meta-analysis identifies signature genes that distinguish microglia from peripheral monocytes/macrophages in health and glioma. Acta Neuropathol Commun 7, 20.). *Trem2* (Gene ID: 83433, a proinflammatory membrane protein likely involved in chronic inflammation, (Dhandapani, R., Neri, M., Bernhard, M. et al. (2022) Sustained Trem2 stabilization accelerates microglia heterogeneity and Aβ pathology in a mouse model of Alzheimer's disease. Cell Rep 39, 110883.), *Cxcl10* (Gene ID: 15945, chemokine (C-X-C motif) ligand 10, potential chemoattractant, has chemokine and heparin binding activity. Normal gene expression in the mouse brain is relatively low compared to other tissues (Yue, F., Cheng, Y., Breschi, A. et al. (2014) A comparative encyclopedia of DNA elements in the mouse genome. Nature 515, 355-364.), however it appears to have a role multiple sclerosis through microglial activation (Tanuma, N., Sakuma, H., Sasaki, A. and Matsumoto, Y. (2006) Chemokine expression by astrocytes plays a role in microglia/macrophage activation and subsequent neurodegeneration in secondary progressive multiple sclerosis. Acta Neuropathol 112, 195-204) and has been implicated in neuropathology in GM2 gangliosidosis (Demir, S. A., Timur, Z. K., Ate 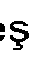 , N., Martinez, L. A. and Seyrantepe, V. (2020) GM2 ganglioside accumulation causes neuroinflammation and behavioral alterations in a mouse model of early onset Tay-Sachs disease. J Neuroinflammation 17,277).

### Example 6- Immunohistochemistry

Unless otherwise stated, the methodology and sample collection were performed as in Example 2.

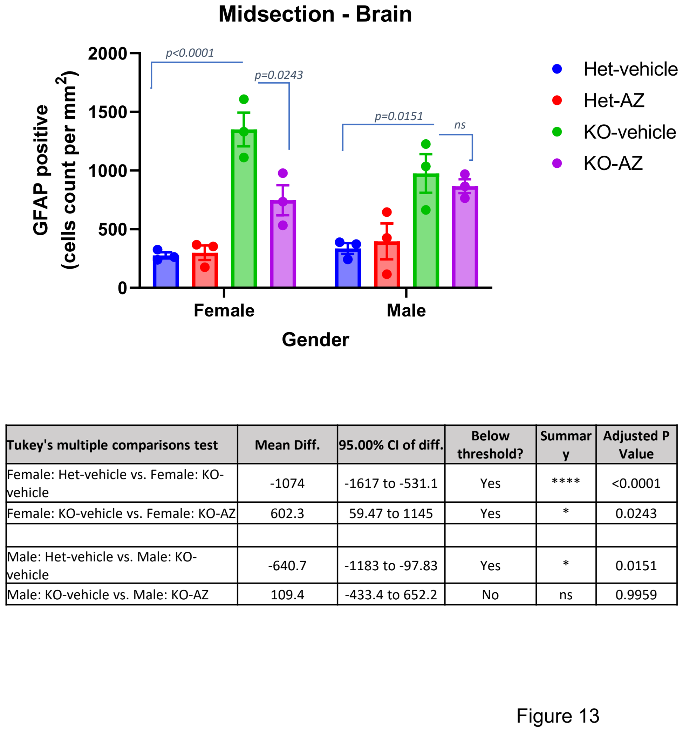

GFAP (Glial fibrillary acidic protein) a class-III intermediate filament protein, is a cell- specific marker that, during the development of the central nervous system, distinguishes astrocytes from other glial cells. Intermediate filaments form networks that provide support and strength to cells. Several molecules of glial fibrillary acidic protein bind together to form the type of intermediate filament found in astroglial cells. Astroglial cells support and nourish cells in the brain and spinal cord. If brain or spinal cord cells are injured through trauma or disease, astroglial cells react by rapidly producing more glial fibrillary acidic protein. Table 6 and Table 7 summarize the immunofluorescence between groups and highlight the remarkable increase in GFAP staining in Hexb -/- (KO) animals compared to both Hexb +/- (Het) treated with vehicle and Hexb -/- treated with AZ-3102, particularly in the midsection of the brain. Graphically, the differences between Hexb -/- mice treated with AZ-3102 treated or vehicle for the midsections is more apparent (Figure 13) with females having a statistical decrease, while male animals show a trend to having a decrease in staining.

**Table 6. Statistics for midsection of the brain.**

| Gender | Het-Vehicle | | Het-AZ-3102 | | KO-Vehicle | | KO-AZ-3102 | |
|---|---|---|---|---|---|---|---|---|
| | Mean | SEM | Mean | SEM | Mean | SEM | Mean | SEM |
| Female | 276.15 | 25.76 | 298.81 | 61.90 | 1350.05 | 143.87 | 747.75 | 128.87 |
| Male | 335.10 | 46.32 | 395.99 | 153.71 | 975.77 | 165.00 | 866.38 | 59.46 |

**Table 7. Statistics for caudal section of the brain.**

| Gender | Het-Vehicle | | Het-AZ-3102 | | KO-Vehicle | | KO-AZ-3102 | |
|---|---|---|---|---|---|---|---|---|
| | Mean | SEM | Mean | SEM | Mean | SEM | Mean | SEM |
| Female | 439.35 | 95.45 | 508.99 | 79.73 | 2329.52 | 101.71 | 1656.84 | 242.78 |
| Male | 574.42 | 42.07 | 425.10 | 82.16 | 1668.21 | 316.09 | 1762.82 | 210.14 |

## Claims

1. A compound of formula (I), 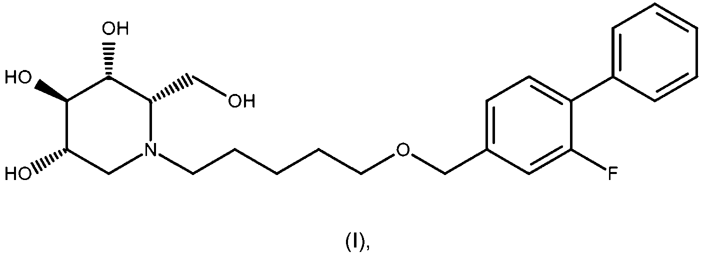 for use in improving motor function of a subject, in particular a subject suffering from GM2 gangliosidosis, specifically Sandhoff disease or Tay-Sachs disease.

2. A compound of formula (I), 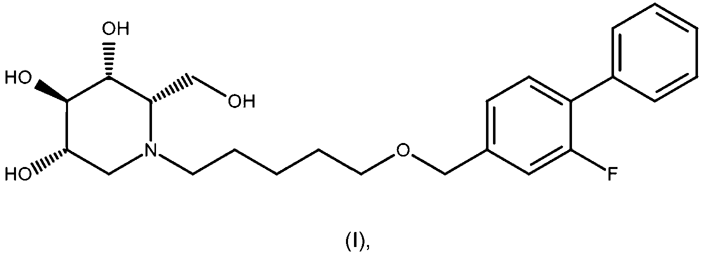 for use in eliminating, alleviating or ameliorating symptoms of GM2 gangliosidosis, in particular Sandhoff disease or Tay-Sachs disease.

3. A compound of formula (I), 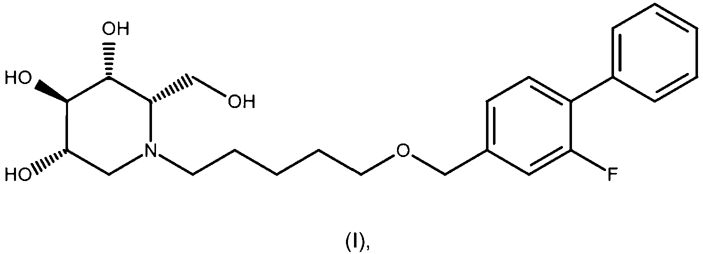 for use in extending life or life expectancy of a subject suffering from GM2 gangliosidosis, in particular Sandhoff disease or Tay-Sachs disease.

4. The compound for use of any one of claims 1-3, wherein the compound is in a crystalline form.

5. The compound for use of claim 4, wherein the crystalline form is a crystalline free base.

6. The compound for use of claim 4 or claim 5, wherein the crystalline form displays a reflection, stated as a 2Θ value, at 17.8 ± 0.2°, in an X-ray powder diffraction pattern, wherein the reflection at 17.8 ± 0.2° is one of the four strongest reflections in the X-ray powder diffraction pattern.

7. The compound for use of claim 6, further displaying one or more reflections, stated as a 2Θ value, at one or more of 4.1 ± 0.2°, 8.3 ± 0.2°, 12.4 ± 0.2°, 13.6 ± 0.2°, 14.5 ± 0.2°, 14.9 ± 0.2°, 15.2 ± 0.2°, 17.2 ± 0.2°, 19.3 ± 0.2°, 21.2 ± 0.2°, 22.4 ± 0.2°, 22.9 ± 0.2° and 23.3 ± 0.2°, in an X-ray powder diffraction pattern.

8. The compound for use of any one of claims 1-7, wherein the compound is administered to the subject at a dosage ranging from about 0.1 mg total/day to about 15 mg total/day.

9. The compound for use of any one of claims 1-8 wherein the compound is administered to the subject together with an anti-inflammatory agent.

10. The compound for use of claim 9, wherein the compound is administered to the subject simultaneously or sequentially with the anti-inflammatory agent.

11. The compound for use of any one of claims 1-10, wherein the compound is administered to the subject daily.

12. The compound for use of any one of claims 1-11, wherein the subject has at least one mutation in a *HEXB* gene and/or shows decreased activity of beta-hexosaminidase A and/or beta-hexosaminidase B.

13. The compound for use of claim 1 or claim 3, wherein the compound provides at least one further therapeutic benefit to the subject, wherein the at least one further therapeutic benefit is selected from improved behaviour, reduced anxiety, improved physical condition, improved balance, reduction of seizures, improved articulation of speech, improved gait, improved swallowing and improved intellectual ability.

14. The compound for use of any one of claims 1-13, wherein the subject is human, preferably the subject is between 28 days and 30 years of age.

15. The compound for use of claim 2, wherein the symptoms of GM2 gangliosidosis include at least one of muscle weakness, decreased movement, loss of motor skills, increased reaction to loud noises, seizures, vision loss, hearing loss, intellectual disability, an eye abnormality, enlarge organs, bone abnormalities, speech difficulties, loss of cognitive function, loss of muscle coordination, and/or psychiatric problems.
